# EUROPÄISCHE PATENTSCHRIFT

(11) **EP 1 179 349 B1**
(45) Veröffentlichungstag und Bekanntmachung des Hinweises auf die Patenterteilung: **15.03.2006**
(21) Anmeldenummer: 00710015.9
(22) Anmeldetag: 11.08.2000
(51) Int. Cl.: A61K 39/39, A61P 31/04, A61P 31/12, A61P 33/00

(54) **W/o Emulsion Adjuvanszuzammensetzungen für Impstoffen**
W/O emulsion adjuvant compositions for vaccines
E/H émulsion adjuvante destinée à des vaccins

(43) Veröffentlichungstag der Anmeldung: 13.02.2002
(73) Patentinhaber: Lohmann Animal Health GmbH & Co. KG, 27472 Cuxhaven (DE)
(72) Erfinder: Hauptmeier, Bernhard, D-53571 Gelnhausen (DE); Lüder, Olaf, 06882 Rosslau (Elbe) (DE); Hellberg, Lutz, 27476 Cuxhaven (DE); Flore, Peter-Harmen, 27478 Cuxhaven-Altenbruch (DE)
(74) Vertreter: Hauck, Graalfs, Wehnert, Döring, Siemons

(56) Entgegenhaltungen:
- EP-A- 0 781 559
- WO-A-93/24147
- WO-A-99/51099
- US-A- 5 885 590
- US-A- 6 022 530

## Beschreibung

Die Erfindung bezieht sich auf Stoffzusammensetzungen und auf diesbezügliche Verwendungen als Adjuvantien für Impfstoffe oder als Impfstoffe.

Nach dem in der Bundesrepublik Deutschland gültigen Arzneimittelgesetz sind Impfstoffe (Vakzine) "Mittel, die Antigene enthalten und die dazu bestimmt sind, bei Menschen oder Tieren zur Erzeugung von spezifischen Abwehr- und Schutzstoffen mittels Impfen angewandt zu werden".

Impfstoffe enthalten als Antikörper insbesondere lebende, abgeschwächte oder tote Erreger (Bakterien oder Viren) bzw. deren Stoffwechselprodukte (Erregergifte/Toxoide) und dienen zur aktiven Bildung von Antikörpern. Im Gegensatz zu dieser aktiven Immunisierung erfolgt bei der passiven Immunisierung eine Injektion von Antiserum, das Antikörper enthält, die durch einen Fremd-Organismus nach Infektion mit dem jeweiligen Erreger gebildet wurden.

Die meisten inaktivierten Impfstoffe fallen unter den Sammelbegriff "Parenteralia" (griechisch: para- = neben und entera = Eingeweide). Als Parenteralia werden systemisch wirkende Stoffe bezeichnet, die unter Umgehung des Magen-Darm-Trakts in den Körper gelangen. Im engeren Sinne umfaßt die Bezeichnung Parenteralia nur die Injektionen und Infusionen (s.c. = subcutan, i.v. = intravenös, i.m. = intramuskulär).

Adjuvans (von lateinisch: adjuvare = Hilfe geben) ist die Bezeichnung für einen Stoff, der die Wirkung eines Impfstoffes steigert. Bei immunchemischen Vorgängen führen Adjuvantien wie z.B. Aluminhydroxide, Kalziumphosphat-Gele oder das Freundsche Adjuvans zusammen mit Antigenen zur verstärkten Bildung von Antikörpern.

Bei Freundschem Adjuvans beruht diese Wirkung unter anderem auf der W/O-Phasenlage, da sich die Wirkstoffe in der wäßrigen inneren Phase der Emulsion befinden. Hierdurch wird die Wirkstofffreigabe verzögert. Eine W/O-Emulsion ist eine "Wasser-in-Öl-Emulsion", d.h. es werden feine Wassertropfen (innere Phase = diskontinuierliche Phase) in Öl (äußere Phase = kontinuierliche Phase) emulgiert.

Das Antigen wird in der Regel in der wäßrigen Phase eingeschlossen. Neben der wäßrigen und der öligen Phase besteht ein modernes W/O-Adjuvant außerdem aus einem oder mehreren Emulgator(en) und Hilfsstoffen zur Verbesserung der Lagerstabilität.

Das "komplette" Freundsche Adjuvans (FCA) ist eine sahneartige W/O-Paraffinöl-Fettemulsion mit abgetöteten Mykobakterien. Es zeigt hervorragende Wirksamkeit, allerdings verursacht es besonders bei größeren Tieren oft große, eiternde Geschwülste. Das "inkomplette" Freundsche Adjuvans (iFA) dagegen enthält keine Mykobakterien.

Für veterinärmedizinische Schutzimpfungen, wo absolute Beschwerdefreiheit vorausgesetzt wird, hat das Freundsche Adjuvans aufgrund dieser Nebenwirkungen keine Chance auf Zulassung. Nachteilig bei dieser Rezeptur ist ebenfalls die sehr geringe Lagerstabilität.

In der Veterinärmedizin werden für verschiedene Zieltierarten und Antigene verschiedene Adjuvantien eingesetzt. Einige Beispiele sind angegeben in P.-P.

Pastoret/J. Blancou/P. Vannier/C. Verschueren, Veterinary Vaccinology, Elsevoa Sciences (1997).

Die US-A-5 885 590 offenbart u.a. Wasser-in-Öl-Emulsionen und Verfahren zu deren Herstellung für zahlreiche Verwendungen, zu denen die Verwendung als Adjuvans für Impfstoffe gehört. Es wird auch die Verwendung von Stabilisatoren vorgeschlagen, insbesondere von Siliciumdioxid.

Davon ausgehend liegt der Erfindung die Aufgabe zugrunde, eine neue Stoffzusammensetzung zu schaffen, die insbesondere für die Verwendung als Adjuvans für einen Impfstoff oder für die Verwendung als Impfstoff geeignet ist und eine gute Verträglichkeit bei parenteraler Applikation aufweist.

Die Aufgabe wird durch eine Stoffzusammensetzung mit den Merkmalen des Anspruches 1 gelöst. Vorteilhafte Ausgestaltungen der Erfindung sind Gegenstand der Unteransprüche.

Die erfindungsgemäße Stoffzusammensetzung ist eine stabile W/O-Emulsion umfassend
- eine disperse wäßrige Phase,
- eine die disperse wäßrige Phase enthaltende kontinuierliche Ölphase auf der Basis eines flüssigen Fettes und/oder eines flüssigen Öles und/oder eines flüssigen Wachses,
- einen Emulgator und
- hydrophobisiertes, hochdisperses Siliciumdioxid mit einem mittleren Teilchendurchmesser im Nanometerbereich als Stabilisator.

Die erfmdungsgemäße Stoffzusammensetzung ist stabil, auf eine Spritze aufziehbar, geeignet für die parenterale Applikation, ähnlich verträglich wie kommerzielle Adjuvantien und ähnlich wirksam wie das inkomplette Freundsche Adjuvans.

Das hydrophobisierte, hochdisperse Siliciumdioxid stabilisiert die Emulsion durch einen Thixothropieeffekt, welcher sich kurz nach der Herstellung einstellt. Die Viskosität der mechanisch unbeanspruchten Emulsion nimmt nach einer gewissen Zeitverzögerung zu. Dieser Zustand ist reversibel, denn ein kurzes Schütteln reicht aus, um die Emulsion in einen fließfahigen Zustand zurückzuversetzen. Dann ist sie ohne weiteres in eine Spritze aufziehbar und parenteral applizierbar. Dies macht die Emulsion insbesondere für einen Einsatz als Adjuvans geeignet. Bemerkenswert ist auch die sehr gute Temperaturstabilität der Emulsion Dies beruht vermutlich auf der erhöhten Gerüststabilität der Siliciumdioxidteilchen, so daß eine mit steigender Temperatur erhöhte Koaleszenzneigung der Tröpfchen durch die Siliciumdioxidteilchen kompensiert wird. In dieser stabilen Stoffzusammensetzung können die Wasserteilchen mit verhältnismäßig großem mittleren Tropfendurchmesser vorliegen (vorzugsweise im Bereich von 5 bis 30 µm), wodurch die einzusetzende Emulgatormenge verringert wird, sich der Herstellungsprozeß vereinfacht (einfachere Mischtechnik) sowie eine niedrigere Viskosität erreicht wird. Bezogen auf 100 ml Emulsion enthält die Stoffzusammensetzung bevorzugt maximal 1,5 g Siliciumdioxid. Der Einsatz des hydrophobisierten, hochdispersen Siliciumdioxid hat zudem positive Auswirkungen auf die Wirksamkeit bei der Verwendung als Adjuvans.

Siliciumdioxid wurde bislang für parenterale Applikationen ausgeschlossen, da in früheren Untersuchungen über toxische Wirkungen (Gewebereizungen, Impfabzesse etc.) berichtet wurde. Überraschenderweise ist dies jedoch bei der erfindungsgemäßen Stoffzusammensetzung mit hydrophobisiertem, hochdispersern Siliciumdioxid nicht der Fall. Dabei ist für die Verträglichkeit von Vorteil, daß äußerst geringe Einsatzmengen des hydrophobisierten, hochdispersen Siliciumdioxids ausreichen, um die gewünschte Stabilisierung zu erzielen. Von Vorteil ist überdies die äußerst einfache Herstellbarkeit der Stoffzusammensetzung.

Die Stoffzusammensetzung kann als Stabilisator ausschließlich hydrophobisiertes, hochdisperses Siliciumdioxid enthalten. Grundsätzlich kann sie aber auch hydrophobisiertes, hochdisperses Siliciumdioxid und Lecithin in Kombination enthalten, um die Vorteile der beiden Stabilisatoren miteinander zu kombinieren. Darüber hinaus ist es möglich, bei dem Einsatz des einen Stabilisators oder der Stabilisatorkombination noch einen oder mehrere weitere Stabilisatoren hinzu zu nehmen.

Die Ölphase kann auf einem einzigen der Stoffe aus den Stoffgruppen der flüssigen Fette, flüssigen Öle oder flüssigen Wachse basieren. Sie kann auch mehrere Stoffe aus einer der Stoffgruppen oder aus mehreren Gruppen in Kombination umfassen. Auch kann sie Stoffe enthalten, die mehreren Stoffgruppen zuzurechnen sind.

Insbesondere für eine Verwendung als Adjuvans bzw. Impfstoff kann die wäßrige Phase der Stoffzusammensetzung auf einer gepufferten Lösung basieren, die aus Wasser für Injektionszwecke und auf den Organismus abgestimmten physiologischen Mineralzusätzen bestehen kann, insbesondere aus einer Phosphatpufferlösung (PBS).

Gemäß einer weiteren Ausgestaltung umfaßt die Stoffzusammensetzung ein Konservierungsmittel, insbesondere im Hinblick auf die Verlängerung der Haltbarkeit für die Aufbewahrung eines Impfstoffes. Bevorzugt ist das Konservierungsmittel im wesentlichen in der wäßrigen Phase enthalten, insbesondere wenn diese der Aufnahme eines Antigens oder eines Wirkstoffes dient.

Die Ölphase kann insbesondere auf einem Neutralöl und/oder einem Paraffinöl und/oder Isopropylmyristat und/oder Isopropylstearat und/oder Isopropylpalmitat und/oder Ethyloleat und/oder Octylpalmitat und/oder Oleyloleat und/oder Hexyllaurat basieren. Zur Charakterisierung der vorerwähnten Stoffe wird insbesondere Bezug genommen auf A. Wade / P.J. Weller, Handbook of Pharmaceutical Excipents, The Pharmaceutical Press London 2^{nd} (1994). Die Charakterisierung des Neutralöls ist dort im Abschnitt "Medium Chain Triglyceride" auf den Seiten 299 bis 301 angegeben. Isopropylmyristat ist dort auf den Seiten 243 bis 244 charakterisiert. Die Untersuchung der Stoffzusammensetzung wurden mit Isopropylmyristat durchgeführt. Der Einsatz der weiteren oben aufgeführten Stoffe läßt vergleichbare Ergebnis erwarten, da es sich um Stoffe mit ähnlichen chemischen Eigenschaften handelt. Die Erfindung schließt sowohl sämtliche Stoffzusammensetzungen ein, die nur eine der vorerwähnten Substanzen enthalten, als auch Stoffzusammensetzungen, die beliebige Kombinationen der vorerwähnten Substanzen enthalten.

Der Emulgator ist bevorzugt im wesentlichen in der Ölphase enthalten. Der Emulgator kann ein bestimmter Stoff oder eine Mischung aus verschiedenen Stoffen sein. Bei dem Emulgator kann es sich insbesondere um eine Polyglycerol undloder um ein Polyglycerid handeln. Bevorzugt kommt als Emulgator Polyglyceryl-2Dipolyhydroxistearat zum Einsatz, gegebenenfalls auch in Kombination mit anderen Emulgatoren.

Das Siliciumdioxid ist bevorzugt im wesentlichen in der Ölphase enthalten. Das Lecithin lagert sich vorzugsweise an der Grenzfläche zwischen der öligen und wäßrigen Phase an. Das hochdisperse Siliciumdioxid weist einen mittleren Teilchendurchmesser im Nanometerbereich auf, d.h. im Bereich von 1 bis zu einigen Hundert Nanometern. Bevorzugt hat das Siliciumdioxid einen mittleren Teilchendurchmesser von 10 bis 20 Nanometer.

Gemäß einer vorteilhaften Weiterbildung ist das Siliciumdioxid an der Kieselsäureoberfläche hydrophobisiert, beispielsweise durch Silanol und Siloxangruppen, die auf der Kieselsäureoberfläche angereichert sind.

Besonders vorteilhaft ist eine Stoffzusammensetzung die bezogen auf etwa 100 ml Emulsion 25 ± 5 ml . Isopropylmyristat, 1,5 ± 1,0 ml Polyglyceryl-2Dipolyhydroxistearat, 75 ± 5 ml wäßrige Phase und 1 ± 0,5 g Siliciumdioxid enthält Ganz besonders vorteilhaft ist eine Stoffzusammensetzung, bei der die Volumen- bzw. Massenanteile die jeweiligen Mittelwerte der vorstehenden Bereiche annimmt. Stoffzusammensetzungen mit den vorerwähnten Merkmalen haben bei den Überprüfungen der:Phasenlage, der Tropfengröße, den Viskositätsbestimmungen, der Aufziehbarkeit auf eine Spritze, der Stabilität des Entmischungsverhaltens, der Verträglichkeit und der Wirksamkeit bei Einsatz als Adjuvans eines Impfstoffes besonders günstige Ergebnisse erzielt

Bevorzugt ist bei den vorerwähnten Stoffzusammensetzungen der mittlere Tropfendurchmesser der Tropfen der wäßrigen Phase im Bereich von 5 bis 30 µm angesiedelt, vorzugsweise bei etwa 15 µm.

Besonders vorteilhaft ist ferner eine Stoffzusammensetzung, die bezogen auf etwa 100 ml Emulsion 25 ± 5 ml Isopropylmyristat, 2,5 ± 1,5 ml Polyglyceryl-2Dipolydroxistearat, 2,5 ± 1,5 ml Phospholipid und 75 ± 5 ml wäßrige Phase enthält. Ganz besonders vorteilhaft ist eine Stoffzusammensetzung, bei der die Volumen-bzw. Massenanteile die jeweiligen Mittelwerte der vorstehenden Bereiche annimmt. Stoffzusammensetzungen mit den vorerwähnten Merkmalen haben bei den Überprüfungen der Phasenlage, der Tropfengröße, den Viskositätsbestimmungen, der Aufziehbarkeit auf eine Spritze, der Stabilität, des Entmischungsverhaltens der Verträglichkeit und der Wirksamkeit bei Einsatz als Adjuvans eines Impfstoffes besonders günstige Ergebnisse erzielt.

Bei der vorerwähnten Stoffzusammensetzung beträgt bevorzugt der mittlere Tropfendurchmesser der wäßrigen Phase etwa 0,5 bis 5 µm.

In dieser Patentanmeldung sind mit "mittlerer Tropfendurchmesser" und "mittlerer Teilchendurchmesser" Durchmesser bezeichnet, die sich bei Tropfen oder Teilchen mit von der Kugelform abweichender Form aus einer arithmetischen Mittelung von zwei oder mehreren verschiedenen Durchmesser ergeben. Bei Tropfen- bzw. Teilchenkollektiven ist als mittlerer Durchmesser der "Sauterdurchmesser" zugrunde gelegt. Soweit dabei Durchmesser von Teilchen zu berücksichtigen sind, deren Form von der Kugelform abweicht, sind diese Durchmesser wiederum gemäß Obigem gemittelt.

Die Stoffzusammensetzung ist vorzugsweise auf eine Kanüle mit einem Durchmesser größer oder gleich 0,5 mm aufziehbar.

Für die Verwendung als Impfstoff umfaßt die wäßrige Phase mindestens ein Antigen. Bevorzugt ersetzt dabei das Antigen einen Volumenanteil der wäßrigen Phase. Mit dieser Vorgabe gelten die obigen Volumen- und Massenanteile auch für den Einsatz mindestens eines Antigens.

Es können insbesondere ein bakterielles, virales oder parasitäres Antigen, ein Pilzantigen, ein Toxoid, ein bakterielles, virales oder parasitäres Spaltantigen, ein DNA-Strang oder -Strangabschnitt oder ein rekombinantes Protein als Antigen zum Einsatz kommen.

Bei dem Antigen kann es sich insbesondere um ein Impfantigen für Nutztiere oder Menschen handeln. Die Ergebnisse der Verträglichkeits- und Wirksamkeitsuntersuchungen lassen eine Eignung der Stoffzusammensetzung als Impfstoff sowohl für eine Vielzahl von Nutztieren als auch für den Menschen erwarten. Nur beispielhaft werden für verschiedene Nutztiere bzw. für den Menschen die folgenden Antigene genannt:

Schwein: Pasteurella multocida, Bordetella bronchiseptica, Erysipelothrix rhusiopathiae, Escherichia coli, Clostridium perfringens, Actinobacillus pleuropneumoniae, Haemophilus parasuis, Streptokokkus suis, Salmonella serovare, Yersinia enterocolitica, Mycoplasma hyopneumoniae, Porcine Reproduction and Respiratory Syndrom Virus, Influenzavirus, Pseudorabies Virus, Procine Circovirus, Picornavirus, Porcine Parvovirus (PPV), Schweinepestvirus.

Geflügel: Escherichia coli, Salmonella servovare, Campylobacter jejuni und coli, Yersinia pseudotuberculosis, Pasteurella multocida, Haemophillus paragalli narum, Bordetella avium, Mycoplasma gallisepticum, Clostridium perfringens, Clostridium botulinum, Aspergillus fumigatus, Candida albicans, Eimeria-Spezies, Virus der Avlären Encephalomyelitis, Virus der infektösen Bursitis, Virus der infektösen Bronchitis, Virus der Newcastle-Disease, Virus der infektösen Laryngotracheitis, Virus der Marek-Disease, Aviäres Pockenvirus, Arthritis/Tenosynovitisvirus, Virus der infektösen Anämie/CAA.

Rind: Rotavirus, Bovine Virusdiarrhoe/Mucosal disease, Parainfluenza-3-Virus, Rinderpestvirus, Adenovirus, Koronavirus, Virus der Enzootischen Rinderleukose, Herpesvirus (IBR/IPV), Salmonella dublin, Salmonella serovare, Clamydia psittaci, Mycoplasma mycoides, Mycoplasma bovis, Streptokokkus agalactiae, Staphylokokkus aureus, Corynebacterium pyogenes, Escherichia coli, Pasteurella multocida, Haemophilus somnus, Brucella abortes, Camphylobacter fetus, Moraxella bovis, Trichophyton verrucosum, MKS-Virus, Pasteurella haemolytica.

Schaf/Ziege: Paramyxovirus (Pest der kleinen Wiederkäuer), Lungenadenomatosevirus der Schafe (Retrovirus), Maedi/Visna-Virus, Virus der ansteckenden Arthritis und Encephalitis der Ziege, Ziegenpockenvirus, Schafpockenvirus, Clamydia psittaci, Mycoplasma agalactiae, Salmonella Serovare, Escherichia coli, Brucella melitensis, Listeria monocytogenes, Pasteurella multocida, Pasteurella haemolytica.

Mensch: Bordetella bronchiseptica, Escherichia coli, Parvovirus, Rotavirus, Pneumovirus, Adenovirus, Retrovirus, Koronavirus, Salmonella Serovare, Hepatitis-A-Virus, Haemophilus influenziae, Klebsiella pneumoniae, Shigella flexeneria, Francisella tularensis, Pasteurella multocida, Hepatitis-B-Viren, Herpes-Viren (z. B. Herpes-simplex-Viren, Enteroviren).

Die Stoffzusammensetzung wird bevorzugt als Adjuvans für einen Impfstoff verwendet, unter Aufnahme mindestens eines Antigens in die wäßrige Phase. Bevorzugt handelt es sich um eine Verwendung als Adjuvans für einen inaktivierten Impfstoff. Weiterhin vorzugsweise als Adjuvans für einen Impfstoff für Nutztiere oder Menschen. Bevorzugt ist die parenterale Applikation.

Die ein Antigen umfassende Stoffzusammensetzung wird bevorzugt als Impfstoff verwendet. Weiterhin vorzugsweise wird sie als inaktivierter Impfstoff verwendet. Schließlich ist eine Verwendung als Impfstoff für Nutztiere oder Menschen besonders vorteilhaft. Bevorzugt ist die parenterale Applikation.

Ausführungsbeispiele der Erfindung sind in den nachfolgenden Abschnitten näher erläutert:
1. Charakterisierung der für die Adjuvantien verwendeten Inhaltsstoffe
2. Adjuvansrezepturen
3. Herstellung der Adjuvansrezepturen
4. Verträglichkeitsprüfungen mit Mäusen, Hühnern und Schweinen
5. Wirksamkeitsprüfungen mit Meerschweinchen (Porcine Parvovirus-PPV) und Kaninchen (rStx-Ile-B-Protein).

Die Ausführurigsbeispiele beziehen sich auf die anliegenden Figuren. Darin zeigen:
- Fig. 1: Schematische Darstellung von Silanolgruppen auf der Aerosiloberfläche der hydrophobierten Varianten R972, R974.
- Fig. 2: Schematische Darstellung der Wechselwirkungen von Aerosilpartikeln miteinander.

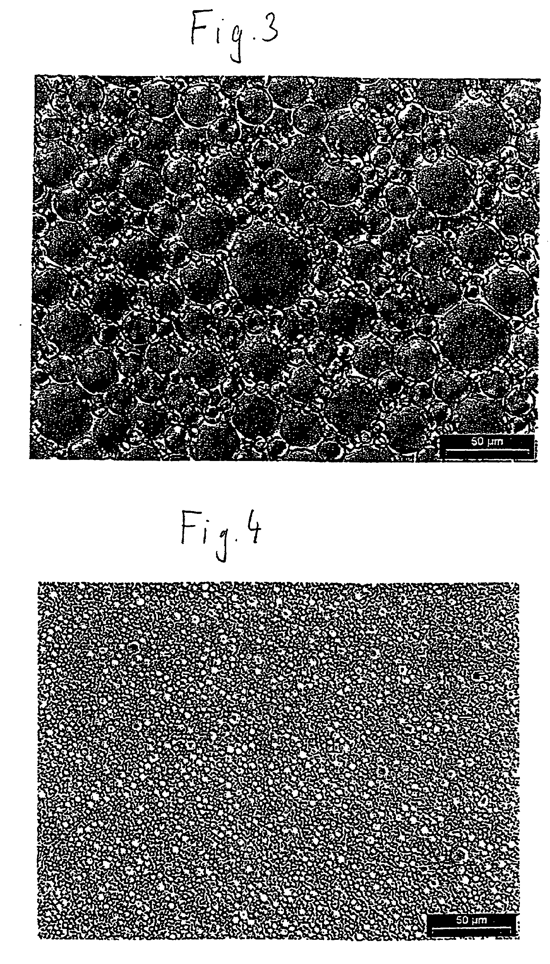

- Fig. 3: Lichtmikroskopische Aufnahme zur Charakterisierung der Placebo W/O Emulsion G 98 V04-101.
- Fig. 4: Lichtmikroskopische Aufnahme zur Charakterisierung der Placebo W/O Emulsion G 99 V09-12.
- Fig. 5: Rühreraufbau im Vertikalschnitt.
- Fig. 6: Aufbau des IKA UTL 25 Inline Ultra Turrax im Längsschnitt.
- Fig. 7: Durchschnittliche Gewichte der Mäuse am Versuchsende.
- Fig. 8: Durchschnittliche Gewichte der Hähne am Versuchsanfang, vor der 2. Impfung und am Versuchsende.
- Fig. 9: Bewertung der Injetionsstellen nach Opitz.
- Fig. 10: PPV-Antikörpertiter im Meerschweinchen nach Impfung mit verschiedenen Impfstoffen.
- Fig. 11: Anti-Stx2e-Antikörpertiter bei Kaninchen nach Impfung mit rHis-StxB2e und verschiedenen Adjuvantien.

### 1. Charakterisierung der für die Adjuvantien verwendeten Inhaltsstoffe

Die Inhaltsstoffe der favorisierten Rezepturen werden in diesem Abschnitt näher beschrieben. Es werden ...Daten aus
den Stoff- und Sicherheitsdatenbiättern der Hersteller aufgeführt.

### 1.1 Isopropylmyristat (Tegosoft® M)

- Funktion:: Ölkomponente
- Hersteller:: z.B. Th. Goldschmidt AG, Essen , Tegosoft® M
- Summenformel:: C₁₇H₃₄O₂
- Molekülstruktur::

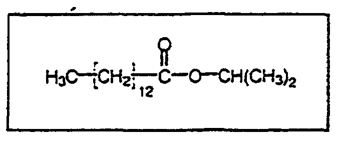

| | | |
|---|---|---|
| Molekulargewicht: | 270,5 | g/mol |
| Farbe: | Farblos, transparent | |
| Geruch: | Geruchlos | |
| Verseifungszahl: | 202-212 | mg KOH/g |
| Säurezahl: | <1 | mg KOH/g |
| Wassergehalt: | <0,1 | % |
| Aggregatzustand: | Flüssig | |
| Dichte (20°C): | 0,85-0,855 | g/ml |
| Viskosität (20°C): | 7-9 | mPa • s |
| Siedepunkt: | 192 | °C |
| Brechungsindex_{20°c}: | 1,434-1,437 | |
| Toxizität: | Keine toxische Wirkung nachweisbar. | |
| Keimzahl: | <10 KBE/ml | |
| Monographie: | DAB 10, britisches Arzneibuch; MRL Status: Annex II | |

### 1.2 Polyglyceryl-2 Dipolyhydroxystearat (Dehymulse® PGPH)

| | | |
|---|---|---|
| Funktion: | W/O-Emulgator | |
| z.B. | Cognis Deutschland GmbH (bis 1998 Carechemicals, Henkel) | |
| Hersteller: | Dehymuls® | PGPH |
| Molekulargewicht: | 3000 | g/mol |
| Aussehen: | Gold-gelb, leicht trüb, viskos | |
| Geruch: | Angenehm herb bis schwach fettig | |
| Verseifungszahl: | 170-190 | mg KOH/g |
| Säurezahl: | <1 , | mg KOH/g |
| HLB-Wert: | 3-5 | |
| Dichte (20°C): | 0,95 | g/ml |
| Viskosität (20°C): | 5500-6000 | mPa • s |
| Siedepunkt: | >180°C | |
| Toxizität: | Es konnte keine toxische Wirkung nachgewiesen werden (LC₅₀>10,000mg/l). | |
| Keimzahl: | <10 KBE/ml | |
| Monographie: | Es existiert derzeit keine Monographie in einem Arzneibuch. Allerdings sind alle Einzelkomponeneten, aus denen der Emulgator zusammengesetzt ist (Glycerin, Stearinsäure, Glycerinester), in Arzneibüchern monographiert. MRL Staus: Die Hauptkomponenten sind mittelkettige Triglyceride, diese sind physiologische (im Körper vorkommende) Substanzen; MRL Status: Annex II. | |

### 1.3 Hydrophobiertes hochdisperses Siliciumdioxid (Aerosil® R974)

| | |
|---|---|
| Funktion: | Stabilisator |
| Hersteller: | z.B. Degussa Hüls AG , Aerosil® R974 |
| Strukturformel: | SiO₂ |

- Primärpartikeiaufbau::

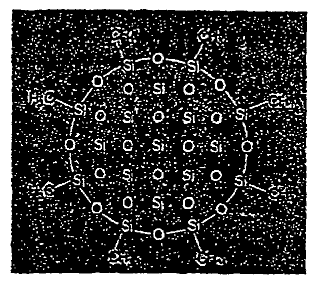

| | | | |
|---|---|---|---|
| Molekulargewicht: | | 60,08 | g/mol |
| Aussehen: | | Weiß, bis fast weißes amorphes Pulver | |
| Geruch: | | Geruchlos | |
| Dichte (20°C): | | 2,2 | g/ml |
| Stampfdichte: | | 50 | g/l |
| Spez. Oberfläche: | | 150-190 | m²/g |
| Trocknungsverlust: | | <0,5 | % |
| C-Gehalt: | | 0,7-1,3 | % |
| SiO₂-Gehalt²⁰: | | >99,8 | % |
| Mittlerer ∅ Primärteitchen: | | 12 | nm |
| Toxizität: | LD₅₀, Ratte > 5000mg/kg, einwandfreie Schleimhautverträglichkeit; es konnten keine histologischen oder pathologischen Veränderungen bei Ratten festgestellt werden, denen über einen Zeitraum von 6 Monaten täglich 500mg Aerosil R 972 oral zugeführt wurde. Es konnte keine kanzerogene oder co-kanzerogene Wirkung festgestellt werden. Aufgrund der amorphen Struktur ist eine Silikose auszuschließen | | |
| Keimzahl: | Aufgrund der Produktstruktur, dem geringen Wassergehalt und dem Herstellungsverfahren, ist Aerosil R 974 mit großer Wahrscheinüchkeit autosteril. | | |

| | | | |
|---|---|---|---|
| ²⁰ Bezogen auf die geglühte Substanz | | | |

- Monographie:: Hochdisperses Siliciumdioxid ist nach EAB allgemein zur oralen und topischen Applikation als pharmazeutischer Hilfsstoff freigegeben.

### Sonstige Anaaben:

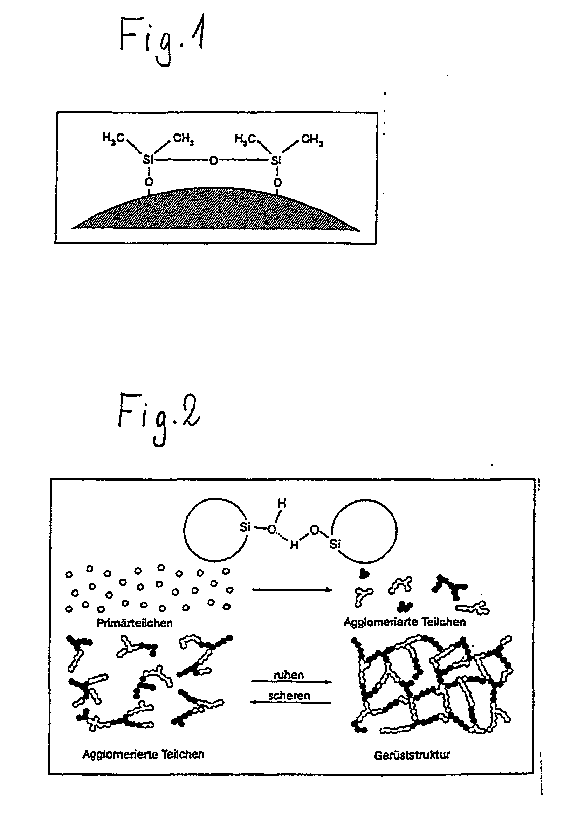

Alle Aerosil-Varianten sind synthetische, hochdisperse Kieselsäuren, die mit dem von Degussa entwickelten Hochtemperaturhydrolyse-Verfahren hergestellt werden. Bei den hydrophobierten Aerosil Varianten sind Silanolgruppen (Si-OH) und Siloxangruppen (Si-O-Si) auf der Kieselsäureoberfläche angereichert (vgl. Fig. 1, Quelle: Degussay AG, Aerosil in Pharmazie und Kosmetik, Heft. Nr. 49, Schriftenreihe Pgimente (1992).

Der Aufbau einer Gerüststruktur sorgt ursächlich für die Entstehung des Thixotropieeffektes beim Einsatz von Aerosil R974 in Öleii.

Der Aufbau von Agglomeraten und einer Gerüststruktur führt zur Thixotropie. Durch Scherkrafteinwirkung kann das Gerüst reversibel zerstört werden (vg1. Fig. 2 - vgl. o.g. Quelle).

### 1.4 Phospholipid (Sternpur® PM)

| | |
|---|---|
| Funktion: | Stabilisator |
| Hersteller: | z . B . Stern Lecithin & Soja GmbH & Co. KG , Sternpur® PM |
| Allgemeiner Aufbau eines Phospholipids: | |

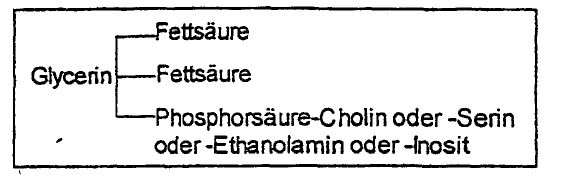

Allgemeine Molekülstruktur eines Phospholipids:

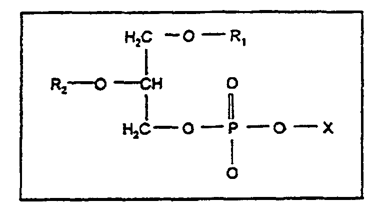

Spezielle Molekülstruktur des Phosphatidyl-Cholin: 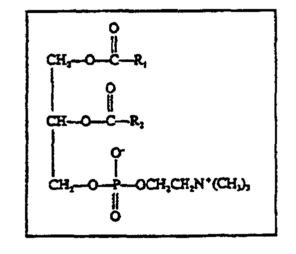
- Herkunft:: Pflanzlich (Soja-Lecithin)
- Aussehen:: Hellgelbes Pulver
- Geruch:: Herb, aromatisch (wie konz. Sojaöl), arteigen

| | | |
|---|---|---|
| Verseifungszahl: | <3 | mg KOH/g |
| Säurezahl: | <35 | mg KOH/g |
| Phospholipidgehalt: | >96 | % |
| Dichte (20°C): | 1,0 | g/cm³ |
| Viskosität (20°C): | - | |
| Schmeizpunkt: | 45-50 | °C |

- Toxizität:: Keine Toxizität nachweisbar; kein ADI-Wert festgesetzt.
- Keimzahl:: Standardware <3000 KBE/g, Pharmaqualität <1000KBE/g
- Monographie:: Stempur PM entspricht den Rechtsvorschriften der EG-Richtlinie 95/2 EG für E322 (Lecithin).

### Sonstige Angaben:

Der Begriff Lecithin umfaßt eine ganz Gruppe sogenannter Phospholipide; im engeren Sinn wird aber häufig nur das Phosphatidyl-Cholin als Lecithin bezeichnet. Die Phospholipide kommen als Zellbausteine in allen tierischen und pflanzlichen Zellen vor.

Allgemein kann gesagt werden, daß je höher der Phosphatidyl-Cholin (PC) Gehalt ist, desto teurer ist auch das Produkt. Man findet Lecithin in größeren Mengen in Eidotter (daher auch der Name: vom griechischen "lekithos"=Dotter). Gewonnen wird es allerdings hauptsächlich aus Sojabohnen.

### 1.5 Phosphatpuffer (PBS)

Der Phosphatpuffer besteht aus Wasser für Injektionszwecke und auf den Organismus abgestimmte physiologische Mineralsalzzusätze. Die Zusammensetzung des Phosphatpuffers ergibt sich aus der Zusammenstellung folgender Komponenten:

| Zusammensetzung Phosphatpuffer (bezogen auf 100ml): | |
|---|---|
| Natriumchlorid | 0,800 g |
| Kaliummchlorid | 0,020 g |
| Dinatriumhydrogenphosphat 2 H₂O | 0,114 g |
| Kaliumdihydrogenphosphat | 0,012 g |
| Wasser für Injekt. (steril) | ad. 100 ml |

| | |
|---|---|
| Dichte (20°C): | 1,003 g/ml |
| Viskosität (20°C): | 1,002 mPa·s |
| pH-Wert (20°C): | 7-7,2 |
| Tonizität | 280-310 mOsm |

### Sonstige Angaben:

Der Mineralsalzzusatz dient in erster Linie der Isotonisierung der wäßrigen Phase im Impfstoff.

Während bei der Herstellung der Placeboemulsionen die wäßrige Phase ausschließlich PBS enthält, besteht die wäßrige Phase eines vollständigen Impfstoffes sowohl aus PBS, als auch aus einem Antigenanteil. Letzterer wird auf die geforderte Wirkstoffkonzentration eingestellt.

Generell ist eine Vermischung der Adjuvantien mit unterschiedlichen Antigenen möglich.

### 1.6 Thiomersal

- Funktion:: Konservierungsmittel
- Strukturformel:: C₉H₉HgNaO₂S
- Molekulargewicht:: 404,81 g/mol
- Molekülstruktur:: 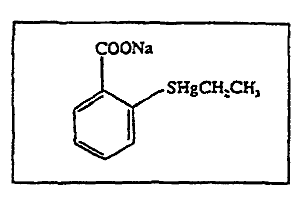
- Herkunft:: Anorganisch
- Aussehen:: Cremefarbenes, kristallines Pulver
- Geruch:: Charakteristisch, arteigen
- Sicherheitsbestimmungen:: Nur unter dem Abzug verarbeiten, nicht einatmen, nicht mit der Haut oder den Schleimhäuten in Berührung kommen lassen.

### Sonstige Angaben:

Das Thiomersal wird in der PBS-Lösung vor dem Emulgieren gelöst. Im o.g. "Handbook of pharmaceutical Excipients" ist bei Injektionslösungen (i.m., i.v., s.c.) eine 0,01%-ige Thiomersalkonzentration als Richtwert vorgeschlagen. Die quecksilberhaltige Verbindung wird seit 1930 für pharmazeutische Zwecke als Konservierungsmittel eingesetzt.

### 2. Adjuvarttrezepturen

### 2.1 Charaktensierung der Rezeptur G 98 V04-101

| Rezeptur. G 98 V04-101: | |
|---|---|
| Isopropylmyristat (Tegosoft M®): | 24,77 ml |
| Polyglyceryl-2 Dipolyhydroxystearat (Dehymuls® PGPH) : | 0,93 ml |
| Phosphatpufferlösung (PBS): | 74,30 ml |
| Hydrophobiertes hochdisperses Siliciumdioxid (Aerosil® R974) | :0,743 g |

D.h. auf 100ml Emulsion werden 0,74 g Aerosil R974 zugegeben. Der Wasseranteil der Emulsion beträgt ~75%, der Ölanteil ~25%.

Wesentliche Merkmale der Rezeptur G 98 V04-101 sind:
■ Stabile W/0 Emulsion mit akzeptabler Viskosität
■ Geringer Entmischungsgrad
■ Phasenverhältnis: W/O ~ 75/25
■ Sehr großer mittlerer Tropfendurchmesser (d_{p,max} > 20µm)
■ Rheologische Eigenschaften: strukturviskos, thixotroph
■ Einsatz von nanoskaligen Feststoffpartikeln ("Nanopartikeln") dient zur Stabilisierung des W/0 Systems mit zusätzlichen positiven Auswirkungen auf die Wirksamkeit des Adjuvans
■ Sehr temperaturstabile Emulsion
■ Sehr einfaches Herstellungsverfahren

Das augenscheinlichste Merkmale der Placebo-Emulsion G 98 V04-101 ist der Thixothropieeffekt, weicher sich kurz nach der Herstellung einstellt. D.h. die Emulsion erscheint nach einer gewissen Standzeit wie "erstarrt". Dieser Zustand ist reversibel, so daß ein kurzes Schütteln ausreicht um das aufgebaute Gerüst aus wescfisetwirkenden Nanopartikeln zu zerstören.

Bei thixothropen Substanzen verringert sich die Viskosität unter der zeitlichen Einwirkung der Scherbeanspruchung. Nach einiger Ruhezeit regeneriert die Flüssigkeit und nähert sich ihrer ursprünglichen Viskosität wieder an. Thixotropie tritt meist zusammen mit Strukturviskosität auf. Ursache für diese Fließanomalie ist der Aufbau von Motekülgerüsten durch Anziehungskräfte.

D.h. auf die Formulierung G98 V04-101 bezogen findet in Abhängigkeit der Zeit eine Verlangsamung der Entmischung statt, die ab einer bestimmten Aerosil R974-Konzentration praktisch zur Erstarrung führt.

Auch das angeführte Merkmal der sehr guten Temperatur Stabilität kann mit dem Thixothropieeffekt erklärt werden: Bei höheren Temperaturen steigt die Neigung des W/O Systems zur Entmischung. Die Stabilisierung durch die beschriebenen Gerüststrukturen wird ebenfalls beschleunigt. Aufgrund der vielbeschriebenen Charakteristika von Nanopartikein ist diese Stabilisierung wiederum schneller als die schnellste destabiliserende Reaktion. Vermutlich wirken sich die zwischenmolekularen Van der Waals Kräfte der Nanopartikel schneller aus als die Gravitationskraft, welche u.a. die Koaleszenz verstärkt.

Weiterhin ist eine Stimulierung des Immunsystems in der Zieltierart erwünscht, um eine verbesserte Immunantwort zu bewirken, ohne inakzeptable Nebenwirkungen, wie Gewebeirritationen, geringere Tageszunahmen, etc. zu erzeugen.

Das erwähnte Merkmal des großen mittleren Tropfendurchmessers wird durch die Fig . 3 verdeutlicht:

Es ist eine typische Tropfen-Größenverteilung dieser Rezeptur zu erkennen. Die Probenoberfläche erscheint plastisch, da mit polarisiertem Licht in einem Phasenkontrastmikroskop gearbeitet wurde. Durch diese Mikroskopiertechnik ist gut zu erkennen, daß die größeren Tropfen durch das aufgelegte Deckglas und die entstehenden Kapillarkräfte zusammengedrückt werden, während kleinere Tropfen weiterhin fast ideale Kugeln bleiben. Insofern wird durch das Auflegen des Deckglases die Tropfen-größenverteilung verfälscht! Dieser systematische Fehler wird bei der späteren Auswertung der Verteilungsfunktion berücksichtigt.

Im Gegensatz zu der Placebo-Formulierung wird bei der Impfstoff-Variante mit PPV-Antigen (IAD PPV-101) ein bestimmter Anteil PBS durch einen äquivalenten Anteil an Antigen-Volumen ersetzt.

### 2.2 Charakterisierung der Rezeptur G 99 V09 12 (mit Phospholipiden)

| Rezeptur G 99 V09-12: (bezogen auf 100ml) | |
|---|---|
| Isopropylmyristat (Tegosoft M®): | 25,00 ml |
| Polyglyceryl-2 Dipolyhydroxystearat (Dehymuls® PGPH) : | 2,5 ml |
| Phospholipid (Stempur PM®): | 2,5 ml |
| Phosphatpufferlösung (PBS): | 70,00 ml |

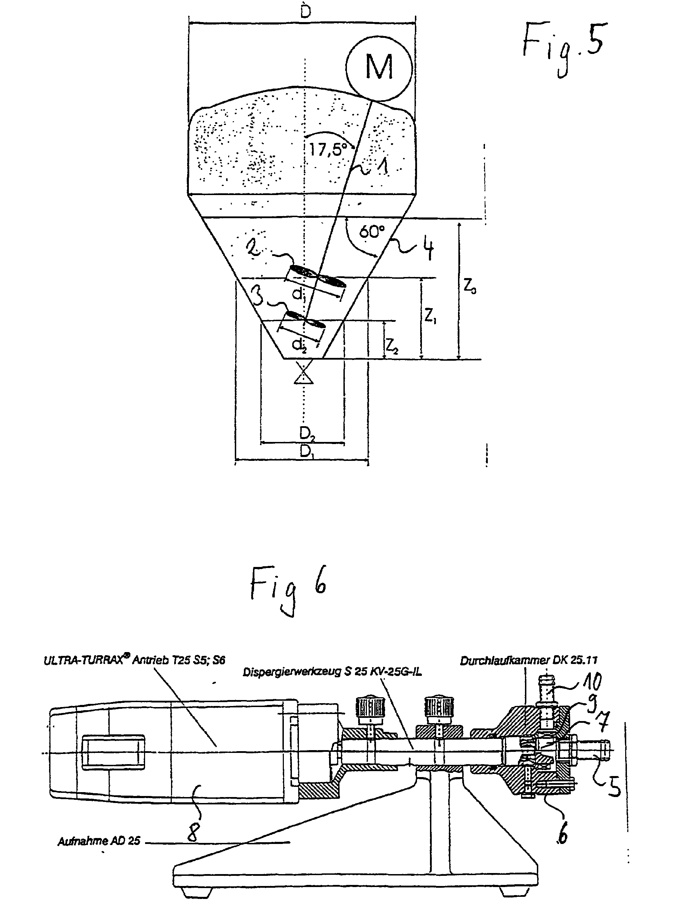

Der wesentlich erhöhte Bedarf an Emulgator ergibt sich aus der vergrößerten inneren Oberfläche. Durch einen kleineren mittleren Tropfendurchmesser wird die zu benetzende Phasengrenzfläche im Vergleich zur Formulierung G 98 V04-101 vervielfacht (vgl. Fig.4). Insgesamt besteht die Formulierung zu 5 % aus oberflächenaktiven Substanzen.

Wesentliche Merkmale der Rezeptur G 99 V09-12 sind:
■ Stabile W/O Emulsion mit etwas erhöhter Viskosität
■ Sehr geringe Entmischung
■ Phasenverhältnis: W/O = 70/25 (+5% Hilfsstoffe)
■ Kleiner mittlerer Tropfendurchmesser
■ Rheologische Eigenschaften: strukturviskos
■ Einsatz von Phospholipiden verbessert die Verträglichkeit
■ Geringer Thixothropieeffekt
■ Herstellungsverfahren nach dem Rotor/Stator-Prinzip

Wenngleich gegenüber der zuvor beschriebenen Rezeptur nur der Hilfsstoff Aerosil R974 gegen Sternpur PM substituiert wurde, handelt es sich bei dieser Formulierung um ein grundlegend verändertes Adjuvans. Es zeigt andere pharmazeutische Eigenschaften und stellt höhere Anforderungen an den verfahrenstechnischen HerstellungsprozeB.

Es ergeben sich bei der Impfstoffvariante IPD PPV-12 praktisch die gleichen Unterschiede wie in der zuvor beschriebenen Vakzine IAD PPV-101. D.h. ein bestimmter PS5-Anteil wird durch konzentriertes Antigen so substituiert, daß der gewünschte Titer pro Impfdosis eingestellt ist.

### 3. Herstellung der Adjuvantrezepturen

### 3.1. Rezeptur G 98 V04-101

Es kommt ein Rührer gemäß Fig. 5 zum Einsatz. Bei diesem sind auf einer Rührwerkswelle 1 zwei dreiblättrige Propellerrührer 2, 3 angebracht.

Der Neigungswinkel der Rührwerkswelle 1 zur Vertikalen und der Wandneigungswinkel des trichterförmigen Rührgefäßes 4 sind eingezeichnet. Die Hauptabmessungen sind hier:
D: Behälterdurchmesser
D₁: Behälterdurchmesser bei Z₁
D₂: Behälterdurchmesser bei Z₂
d₁: Durchmesser großer Propeller
d₂: Durchmesser kleiner Propeller
Z₀: Höhe, Flüssigkeitsoberfläche
Z₁: mittlere Höhe großer Propeller
Z₂: mittlere Höhe kleiner Propeller.

In der folgenden Tabelle sind die gewählten Abmessungen angegeben:

| Bezeichnung | Abmessung / [m] |
|---|---|
| D | 0,200 |
| D₁ | 0,111 |
| D₂ | 0,080 |
| d₁ | 0,043 |
| d₂ | 0,031 |
| Z₀ | 0,130 |
| Z₁ | 0,065 |
| Z₂ | 0,037 |

Die Rohstoffe für die Herstellung der Emulsion G 98 V 04-101, die davon eingesetzten Mengen und die mit dem zuvor beschriebenen Rührer durchzuführenden Herstellungsschritte sind der folgenden Herstellungsbeschreibung entnehmbar:

### W/O Emulsion Placebo G98 V04-101

### Ansatzgröße : 104,75g = 107,46ml

### Zusammensetzung: gemäß H_1:

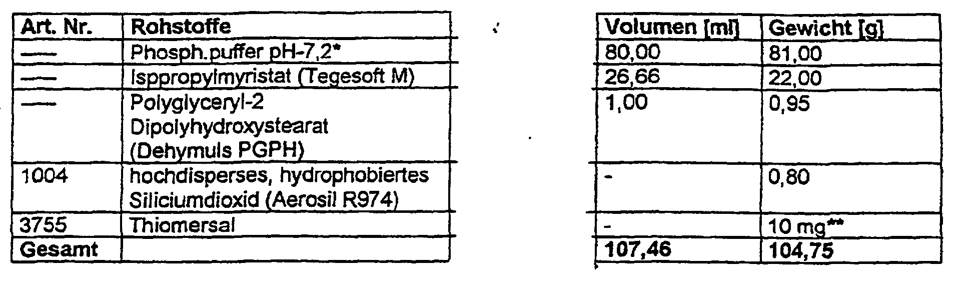

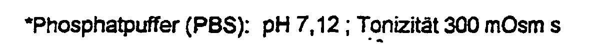

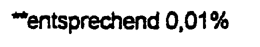

### Herstellungsbeschreibung :

### Teilansatz I

In lsopropylmyristat wird Dehymuls PGPH unter Rühren homogenisiert. Die Mischung wird über einen 0,2 µm Filter steril filtriert

### Teilansatz II

Im bereits sterilem PBS wird Thiomersal gelöst und zur das Ansatzgefäß wird geschwenkt.

### Teilansatz II wird bei laufendem Flügelrührer in Teilansatz 1 gegeben.

Anschließend wird die Emulsion 3 Min. bei 600 min⁻¹ weiter mit dem Flügelrührer dispergiert

### IPK der Vaccine: Phasenlage W/O Emulsion - Färbetest

### Konfektionierung:

In 2x50 ml Klarglasvials, steril HK 1 (3891) mit Brombutytstopfen, steril (3872) und Alukappe (3847) Bei kurzer Zeit zwischen Herstellung und Verabreichung ist eine Lagerung bei Raumtemperatur unter Uchtabschiuß optimal. Bei längerer Lagerzeit sollte bei 5°C gelagert werden.

1xRückstellmuster in 50 ml Klarglasvial bei 5°C in der Kühlkammer

### Bemerkung:

Die Herstellung erfolgte aseptisch unter LF im Sterillabor mit heißluftsterilisiertem Equipment und Primärpackmittel.

| | | | |
|---|---|---|---|
| Merck | Natriumchlorid | # K9107 3504 806 | 0,800 g |
| Merck | Kaliummchlorid | # K23982 436 724 | 0,020 g |
| Merck | Dinatriumhydrogenphosphat 2 H₂O | # K22184980 538 | 0,114g |
| R de H³¹ | Kaliumdihydrogenphosphat | # 73320 | 0,012 g |
| 1235 | Wasser für Injekt. | - | ad.100 ml |

| | | | |
|---|---|---|---|
| ³¹ Riedel de Haen | | | |

### 3.2 Rezeptur G 99 V 09-12

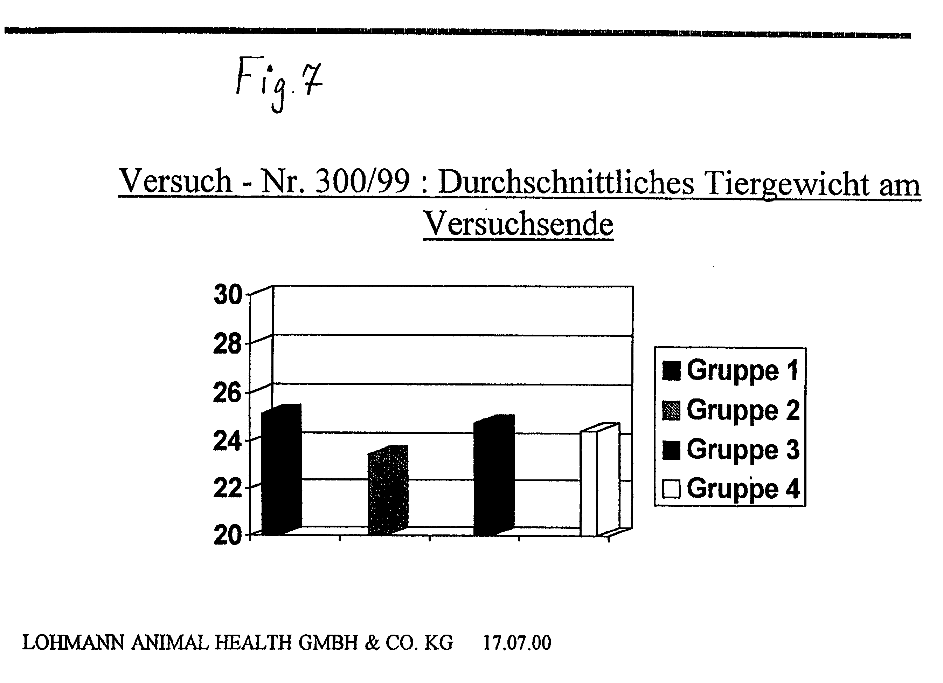

Bei der Herstellung dieser Rezeptur werden wesentlich höhere Scherkräfte für die Erzeugung kleinerer Tropfen eingesetzt. Hierzu ist ein zusätzliches Inline-Rotor-Stator-Dispergierwerkzeug in den Aufbau zur Herstellung der Rezeptur integriert. Es handelt sich dabei um das Dispergierwerkzeug IKA UTL 25 Inline Ultra Turrax der Firma IKA. Dieses ist in der Fig. 6 gezeigt. Die Zuführung des Produktes erfolgt zentral bei 5 in einen Mischkopf 6. Dieser enthält einen Rotor 7, der mittels eines Motors 8 antreibbar ist. Das Produkt wird durch den Rotor 7 beschleunigt, durch Statorschlitze 9 gedrückt und am Umfang aus einem Auslaß 10 ausgetragen.

Die technischen Daten dieses Dispergierwerkzeuges sind in der folgenden Tabelle angegeben:

| Technische Daten IKA UTL 25 Inline Ultra Turrax: | |
|---|---|
| Inline Werkzeuggröße: | 17 mm |
| Rezirkulationsleitung: | DN10 |
| Rotor: | G 20x2 |
| Stator: | G 30x2 |
| Antrieb: | Drehstrommotor |
| Betriebsspannung U: | 240 V ± 10% |
| Antriebsleistung: | 190 W |
| Drehzahl: | 8000-24000min⁻¹, stufenlos verstellbar |
| Zusatzausstattung: | Bypass-Leitung zum Reinigen des Gerätes |

Das Inline-Dispergierwerkzeug ist mit seiner Zuführung an dem Trichterauslauf des Rührergefäßes 4 gemäß Fig. 5 verbunden und vom Auslaß 10 am Umfang des Mischkopfes 6 führt wiederum eine Rückführleitung in das Rührergefäß 4 zurück. Hierdurch wird über das Rührergefäß 4 ein kontinuierlicher Kreislauf durch die Mischkammer im Mischkopf 6 ermöglicht. Aufgrund der eingeschränkten Pumpeigenschaften des Inline-Ultra-Turrax ist es allerdings notwendig, die Mischkammer annähernd auf das Niveau des Produkt-Flüssigkeitsspiegels im Rührgefäß 4 zu bringen.

In der nachfolgenden Herstellungsbeschreibung sind die für die Herstellung der Emulsion G 99 V 09-12 eingesetzten Rohstoffe, deren Mengen und die mit dem zuvor beschriebenen Aufbau durchzuführenden Herstellungsschritte im einzelnen angegeben:

### W/O Emulsion Placebo G99 V09-12

### Ansatzgröße: 96,35g = 100,00ml

### Zusammensetzung: gemäß H_212

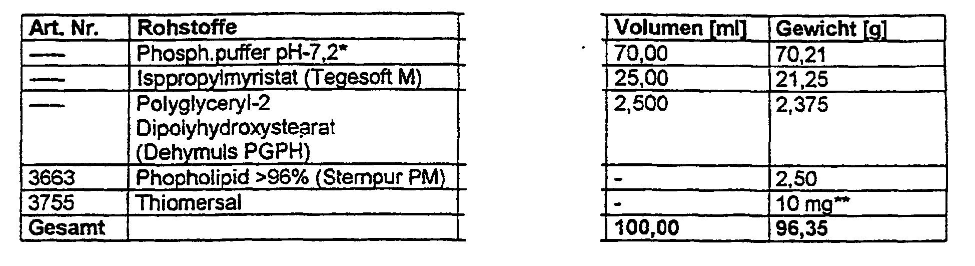

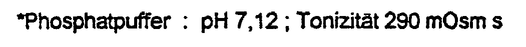

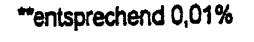

### Herstellungsbeschreibung :

### Teilansatz I

In Isopropylmyristat wird Dehymuls PGPH unter Rühren 2-3 Minuten homogenisiert. Dann wird das Stempur PM Pulver zugegeben und unter ständigem Rühren auf einem beheizbaren Magnetrührer bei 50° C gelöst Die Lösung wird über einen 0,2µm Filter steril filtriert.

### Teilansatz 11

Im bereits sterilfiltrierten PBS wird Thiomersal gelöst und im Ansatzgefäß geschwenkt.

### Teilansatz II wird bei laufendem Rührer in Teilansatz I gegeben. Anschließend wird die Emulsion

3 Min. bei 600min⁻¹ mit dem Rührwerk dispergiert Die Feindispergierung erfolgt bei 18000-24000min^{-1 32} 5 Min. mit dem Ultra-Turrax.

### IPK der Vaccine: Phasenlage W/O Emulsion - Färbetest

### Konfektionierung

In 2 x 50 ml Klarglasvials, steril HK 1 (3891) mit Brombutylstopfen, steril (3872) und Alukappe(3847) Bei kurzer Zeit zwischen Herstellung und Verabreichung ist eine Lagerung bei Raumtemperatur unter Lichtabsdiluß optimal. Bei längeren Lagerzeiten sollte bei 5°C gelagert werden.

1xRückstellmuster in 50 ml Klarglasvial bei 5°C in der Kühlkammer

### Bemerkung

Die Herstellung erfolgte aseptisch unter LF im Sterillabor mit heißluftsterilisiertem Equipment und Primärpackmittel.

| | | | |
|---|---|---|---|
| Merck | Natriumchlorid | # K9107 3504 806 | 0,800 g |
| Merck | Kaliummchlorid | # K23982 436 724 | 0,020 g |
| Merck | Dinatriumhydragenphosphat 2 H₂O | # K22184980 538 | 0,114 g |
| R de H | Kaliumdihydrogenphosphat | # 73320 | 0,012 g |
| 1235 | Wasser für lnjekt | - | ad.100 ml |

| | | | |
|---|---|---|---|
| ³² Bei 18mm Rotor mit ca. 14000 Upm, bei erwünschter, höherer Feinheit der Emulsion bei 24000 UPM. | | | |

### 4.1 Verträglichkeitsprüfungen mit Mäusen

### Versuch - Nr. 300/99

### 1. Titel: Versuch-Nr. 300/99 Untersuchung der Verträglichkeit eines neuen Adjuvants an Mäusen

### 2. Detailierte Beschreibung des Versuches

Methode : - randomisierter Versuch
   - Nachweis der Verträglichkeit an der Labormaus mittels klinischer Beobachtung (HAH)
Material : - Impfstoffpräparationen
**Tiere**: - SPF-Mäuse (Charles River)
   Name: NMRI, weiblich
   Anzahl : 42
   Alter : 17-20g
Frei von relevanten Keimen : SPF-Zertifikat
Vakzinationsdosis : 0,5 ml / Tier
Applikation: s. c.

- Beobachtungszeit :: 10 Tage post vacc.
- Bewertungskriterium : -: tägliches Erfassen der gesunden, kranken und toten Tiere - Registrierung des Körpergewichtes am Ende der Beobachtungszeit

### 3. Kontrolltiere

10 Mäusen wird jeweils auf die gleiche Art und Weise PBS verabreicht.

### 4. Ergebnisse

| **Gruppen- Nr.** | **Impfstoff** | **Antigengehalt (PPV)** | **Adjuvant** | **Anzahl der Mäuse** | **durchschn. Körpergewicht in g** | **lokale u./o. systemische Reaktionen** |
|---|---|---|---|---|---|---|
| 1 | IAD PPV 101 (G98 V04-101/PPV) | 5120 HAE | LAHneu | 12 | 25,15 | keine |
| 2 | Placebo (G98 V04-101) | | LAHneu | 10 | 23,44 | keine |
| 3 | VM 010697 | 5120 HAE > 50 U.I E.rh. | AL-hydroxide | 10 | 24,76 | keine |
| 4 | PBS | - | - | 10 | 25,44 | keine |

Die Einzelgewichte der Mäuse sind im Anhang 1 vermerkt.

Die Beurteilung der Injektionsstelle ist in der Anlage 2 dargestellt.

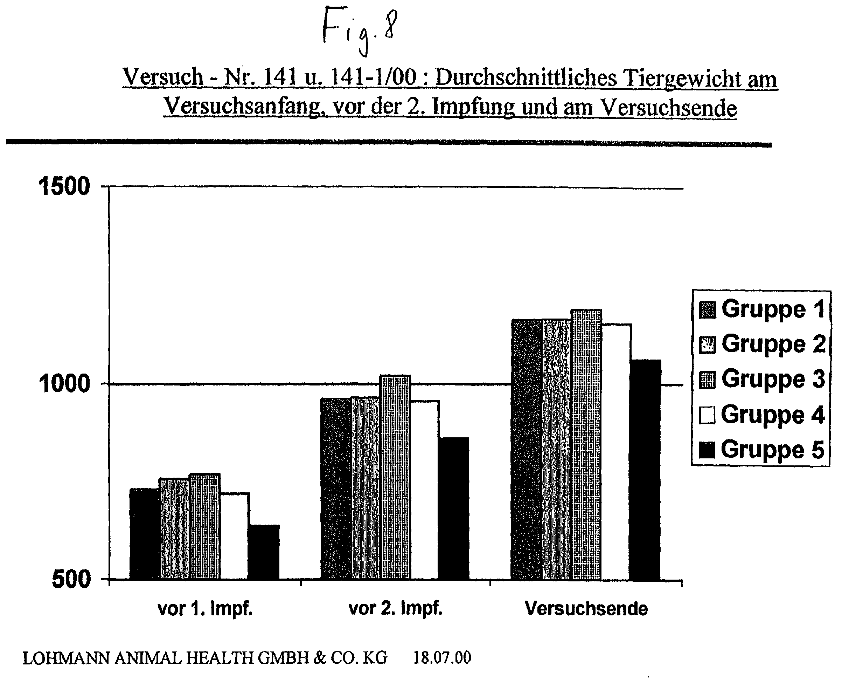

Fig. 7 zeigt die durchschnittlichen Tiergewichte am Versuchsende

### 5. Unerwünschte Nebenwirkungen

- keine

### 6. Während des Versuches verabreichte Medikamente

- keine

### 7. Diskussion der Ergebnisse

Eine s.c. Applikation der verschiedenen Impfstoffmuster an Mäusen führte zu keinen äußerlich sicht- oder palpierbaren Veränderungen an den Injektionsstellen.

Bei der Sektion der Tiere am Versuchsende wurden bei den Tieren der Gruppen 1 und 2 kuglige bis ovale, weiße, trübe Einschlüsse an den Injektionsstellen nachgewiesen. Diese Kapseln ließen sich öffnen und waren gefüllt mit einer gelartigen Flüssigkeit, die sich bei den weiteren Untersuchungen als Impfstoffreste (w/o - Emulsion) erwiesen. So ist anzunehmen, dass es in der Gruppe 1 (Gruppe 2 war eine Placebogruppe) zu einer verzögerten Antigenfreisetzung kommt.
Systemische Reaktionen an den Probanden wurden nicht beobachtet. Die ermittelten Körpergewichte am Ende der Beobachtungszeit zeigen im Gruppendurchschnitt keine signifikanten Unterschiede.
Somit wird eingeschätzt, dass das neu entwickelte Adjuvant nach s.c. Verabreichung für die Weiße Labormaus sicher und verträglich ist.

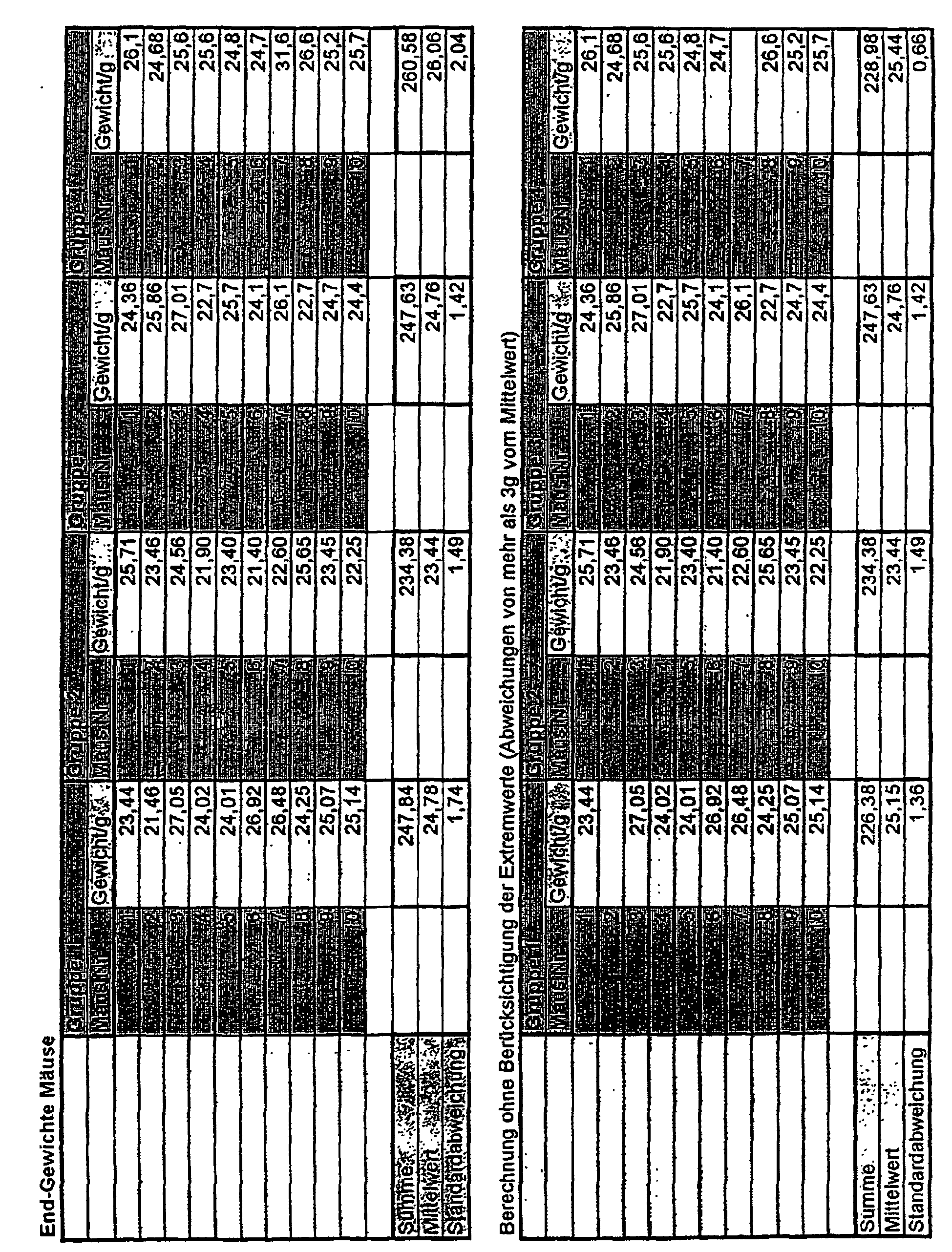

3

**Tabelle Nr. 6: Versuch Nr. 300/99; Begutachtung der Injektionsstelle bei Versuchsende**

| Gruppe Nr. | Befund (systemisch, makroskopisch) | Befund (lokal) |
|---|---|---|
| 1 | o.B. | Kugelige, bis ovale weißlich trübe Einschlüsse an der Injektionsstelle (Kapseln) mit ∅ = 4-5mm; Ein Extremfall mit Kapsel ∅ = 10mm (Diese Probe wurde zwecks mikroskopieren entnommen); Kapseln lassen sich schlechter öffnen als bei Gruppe 2 Leber, Nieren, Milz, etc. o.B. |
| 2 | o.B. | Kugelige, bis ovale weiße, trübe Einschlüsse an der Injektionsstelle (Kapseln) mit ∅ = 3-4mm; Zwei Extremfälle mit Kapsel ∅ = 8mm; Die Kapseln lassen sich leicht öffnen, gelartige Flüssigkeit im Inneren; Leber, Nieren, Milz, etc. o.B. |
| 3 | o.B. | Flächige, relativ fest weiße Einschlüsse an der Injektionsstelle Kapseln mit ∅ = 3-4mm; Zwei Extremfälle mit Kapsel ∅ = 10mm; In der geöffneten Kapsel sind starke Verfestigungen, bzw. Kristattartige Gebilde zu erkennen. Leber, Nieren, Milz etc. o.B. |
| 4 | o.B. | o.B.; Leber, Nieren, Milz, etc. o.B. |

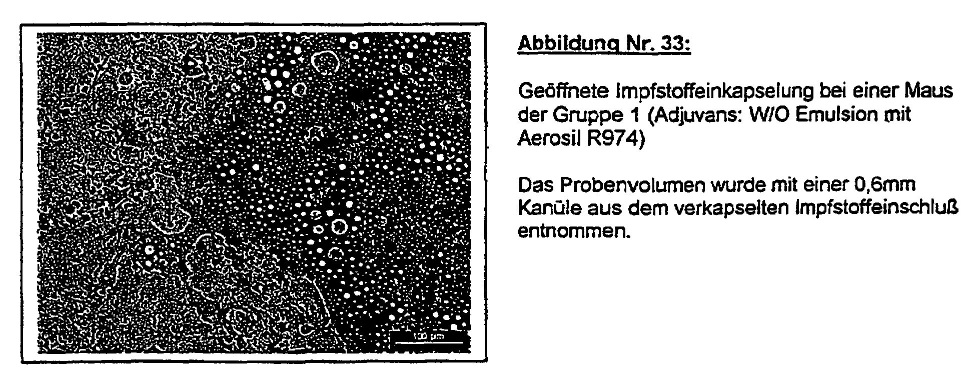

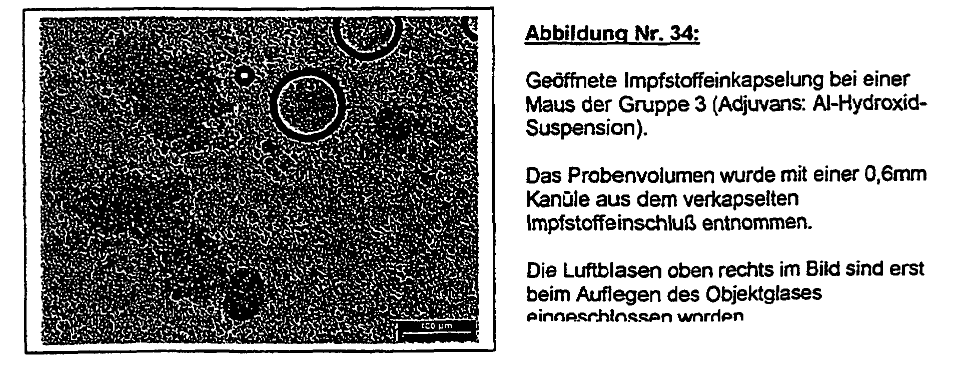

### 4.2 Verträglichkeitsprüfungen mit Hähnen

### Versuch - Nr. 141/00 und 141-1/00

### 1. Titel : Versuch-Nr. 141/00 und 141-1/00 Verträglichkeitsprüfung von 3 Placebochargen in 10 Wochen alten SPF-Hähnen

### 2. Detailierte Beschreibung des Versuches

Methode: - randomisierter Versuch
- Nachweis der Verträglichkeit am SPF-Hähnen mittels klinischer Beobachtung und pathologisch-anatomischer Beurteilung der Injektionsstellen

Material : - Placebopräparationen
- Impfstoff" ND Vac p.i ", Chargen-Nr. : 9065500

**Tiere**: - SPF-Hähne

| | |
|---|---|
| Rasse : | Lohmann White |
| Anzahl: | 30 |
| Alter: | 10 Wochen |

Frei von relevanten Keimen: SPF-Zertifikat
Vakzinationsdosis : 1. Applikation : 0,5 ml / Tier (rechte Brustmuskulatur)
2. Applikation: 1,5 ml / Tier (linke Brustmuskulatur)

Applikation: i.m.

Immunisierung: 2 x im Abstand von 14 Tagen -
- Beobachtungszeit :: 11 Tage post vacc.

| | | |
|---|---|---|
| Bewertungskriterium : | 1. - Bestimmung Körpergewicht - | A) vor 1. Impfung |
| | | B) vor 2. Impfung |
| | | C) am Versuchsende |
| | 2. - tägliche klinische Kontrolle (Bewertung Allgemeinbefinden) | |
| | 3. - lokale Kontrolle der Impfstelle am Versuchsende (Sektion der Tiere und Bewertung der Injektionsstelle nach "Opitz") | |

### 3. Kontrolltiere

10 SPF-Hähnen (ohne Injektion).

### 4. Ergebnisse

| **Gruppen -Nr.** | **Anzahl der Tiere** | **Injektionsmaterial** | **Körpergewicht vor 1. Applikation** | **Körpergewicht vor 2. Applikation-** | **Körpergewicht 14 Tage 2. Applikation*** | **Bewertung der Injektionsstellen (Versuchsende)**** |
|---|---|---|---|---|---|---|
| 1 | 6 | G98 V04-101 (Aerosil hitzesterilisiert) | 732,83 | 963,00 | 1161,50 | 0,50 |
| 2 | 6 | G98 V04-101 (Aerosil sterilfiltriert) | 759,50 | 966,50 | 1163,83 | 0,80 |
| 3 | 6*** | G98 V09-12 (Phospholipide) | 770,33 | 1020,40 | 1188,80 | 0,80 |
| 4 | 6 | ND Vac p.i., Ca-Nr. 9065500 | 722,50 | 957,00 | 1153,00 | 1,17 |
| 5 | 6 | - | 640,33 | 862,50 | 1060,50 | 0,00 |

| | | | | | | |
|---|---|---|---|---|---|---|
| * dudischmädiches Körpergewicht in Gramm | | | | | | |
| ** Bewertung nach Opitz (Anhang1) | | | | | | |
| *** ein Tier durch Karmibalismus am 11. Versuchstag verlorer | | | | | | |

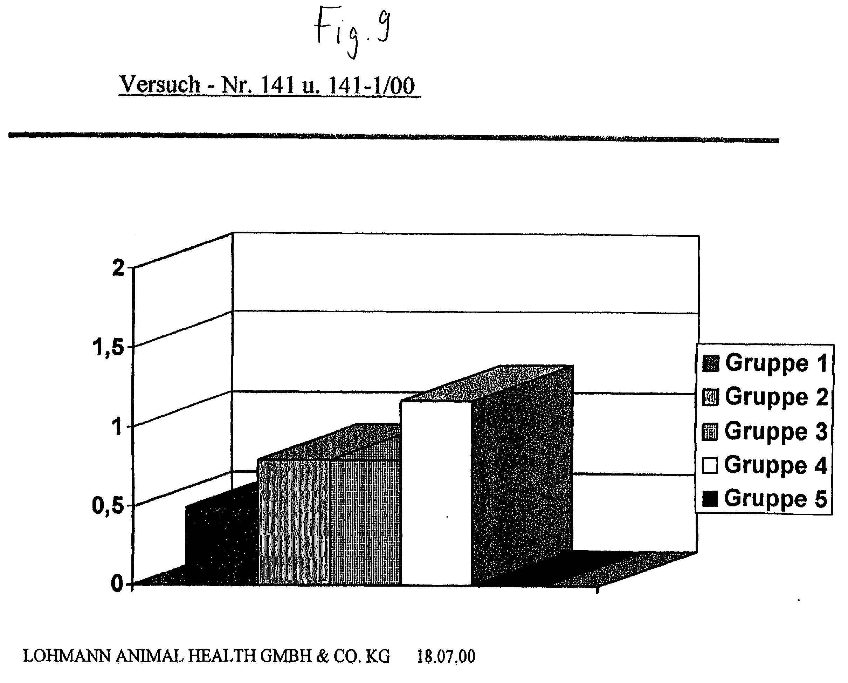

Fig. 8 zeigt die durchschnittlichen Tiergewichte am Versuchsanfang, vor der 2. Impfung und am Versuchsende.

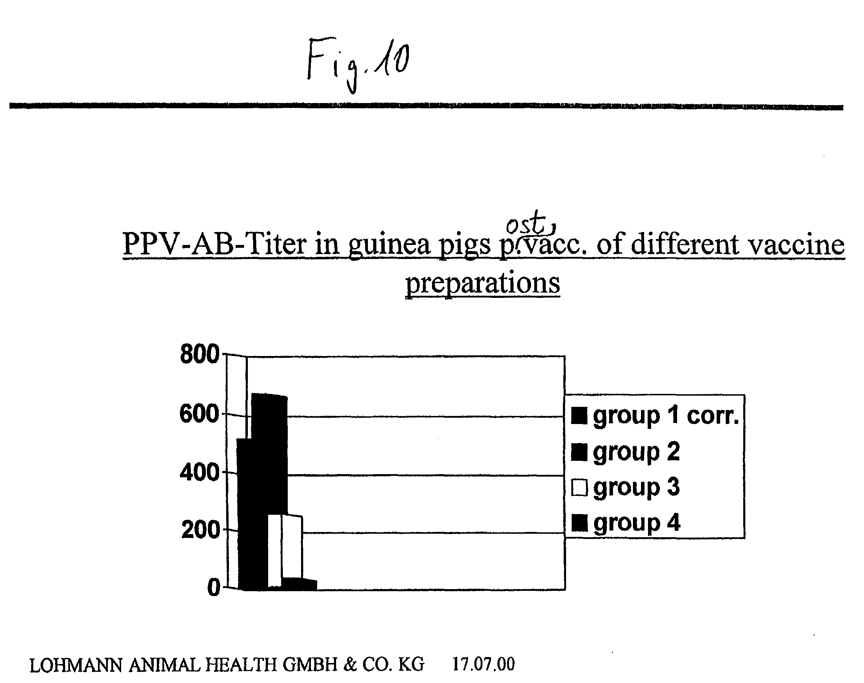

Fig. 9 zeigt die Bewertung der Injetionsstellen nach Opitz

### 5. Unerwünschte Nebenwirkungen

- keine

### 6. Während des Versuches verabreichte Medikamente

- keine

### 7. Diskussion der Ergebnisse

Die Applikation der Placeboformulierungen führte bei keinem der Versuchstiere zu einer Beeinträchtigung des Allgemeinbefindens. Ebenfalls wurden bei keinem Tier lokale Reaktionen an den Injektionsstellen beobachtet (visuelle Beurteilung und Palpation zum Zeitpunkt der 2. Applikation sowie am Versuchsende).
Auch in der Gewichtsentwicklung wurden keine signifikanten Unterschiede zwischen den Gruppen am Versuchsende (14 Tage nach der 2. Applikation) festgestellt. Unterschiede gab es am Versuchsende bei der Bewertung der Injektionsstellen. Dabei zeigten die Tiere der Gruppe, die mit der kommerziellen Vergleichsvakzine geimpft wurden, die größten lokalen Gewebsveränderungen. Die beste Gewebsverträglichkeit demonstrierten die Tiere, die mit der Placebaformulierung G98 V04-101 (Aerosil hitzesterilisiert) vakziniert wurden.

Insgesamt wird eingeschätzt, dass die Formulierungen G98 V04-101 (Aerosil hitzesterilisiert), G98 V04-101 (Aerosil sterilfiltriert) und G98 V09-12 (Phospholipide) bei einer zweimaligen i.m Applikation (0,5 ml/ID und 1,5 ml/ID) in 10 Wochen alten SPF-Hähnen sicher und verträglich sind.

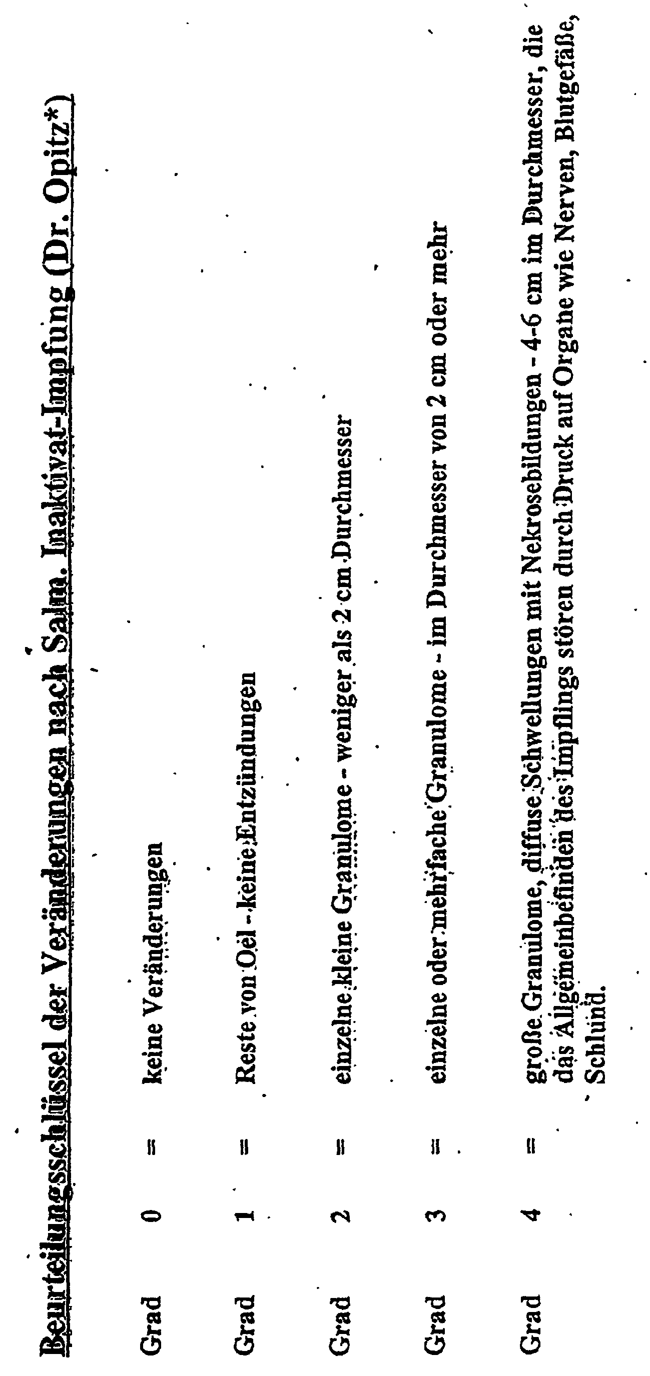

### 4.3 Verträglichkeitsprüfungen mit Schweinen

### Versuch -Nr. : W/O-Adj 01/99

### 1. Titel:

Versuch-Nr. W/O - Adj 01/99
Untersuchung der Verträglichkeit einer neues Adjuvans in Absatzferkeln

### 2. Versuchsmetodik:

Methode : randomisierter Versuch
Versuchsdauer : Dauer der Beobachtung : 4 Wochen

### 3. Tiere

Spezies : Schweine
Typ : Absatzferkel
Sex : weiblich / männlich
Alter am Versuchsbeginn : 5 Wochen
Immunitätsstatus am Versuchsbeginn : gesunde Tiere, kein SPF-Status

### Haltungsbedingungen :

- Gruppenhaltung

### Züchtung / Fütterung / Wasserversorgung / Futterzusätze :

- Züchtung : Deutsche Landrasse x Deutsches Pietrain
- Futter : Läuferfutter
- Wasserversorgung : Trinkwasser
- Futterzusätze : keine

### 4. Parameter der Applikation

Vaccinechargens: A) PPV-Mono-Antigen-bulk 02 0699 ("IAD PPV 101"/98 V04 - 101/PPV)
B) PPV-Mono-vaccine 01 0599

Konzentration der aktive Bestandteile: A) PPV-Mono-Antigen-Bulk 02 0699 -5120HA/ID
B) PPV-Mono-vaccine 01 0599 - 10240 HA / ID

Adjuvantien : A) PPV + G98 V04-101 (W/O-emulsion = IAD PPV 101)
B) PPV + Al-hydroxid

Impfdosis : 1 ID / animal (1 ID = 2 ml)
Impfung: 2 x im Abstand von 14 Tagen
Applikationsweg : parenteral
Applikationstyp : i.m.
Vorbehandlung der Vakzine : -
Vorbehandlung der Tiere : -
Anzahl der vakzinierten Tiere : 4
Anzahl der Kontrolltiere (nicht geimpft) : -

### 5. Methoden der Untersuchung

### 5.1. Palpation der Injektionsstellen

### 5.2. Messung der rektalen Körpertemperatur

- am Tag vor der Injektion
- unmittelbar nach der Injektionern
- 1. und 2. Tag nach der Injektion

### 5.3. Beurteilung des Allgemeinzustandes und der Futteraufnahme der Absatzferkel

### 6. Versuchsverlauf

### 6.1. Unerwünschte Nebenwirkungen der Impfungen

- keine

### 6.2. Eiafluß anderer Faktoren

Anzahl der Tiere, die vor dem Versuchsende ausfielen :
- keine

Auftreten von Erkrankungen während des Versuches :
- keine

Behandlung Tiere mit anderen Arzneimitteln während des Versuches :
- keine

### 7. Ergebnisse

### 7.1. Palpation der Injektionsstellen

| Tier-Nr. | Vakzine | Palpation der Injektionsstelle | | | | | | | | |
|---|---|---|---|---|---|---|---|---|---|---|
| | | 25.08. | 26.08. | 27.08. | 28.08. | 06.09. | 07.09. | 08.09. | 20.09. | 21.09. |
| | | Datum der Injektionen | | | | | | | | |
| | | 24.08. | | | | | 07.09. | | | |
| I | IAD PPV **101** | 0 | 0 | 0 | 0 | 0 | 0 | 0 | 0 | 0 |
| 2 | | 0 | 0 | 0 | 0 | 0 | 0 | 0 | 0 | 0 |
| 3 | 01 0599 | 0 | 0 | 0 | 0 | 0 | 0 | 0 | 0 | 0 |
| 4 | | 0 | 0 | 0 | 0 | 0 | 0 | 0 | 0 | 0 |

0 .... keine lokalen Reaktionen
1.... leichte lokale Reaktionen
2 .... mittlere lokale Reaktionen
3 .... schwere lokale Reaktionen

### 7.2. Messung der rektalen Körpertemperatur

| Tier-Nr. | Vakzine | Datum der rektalen Körpertemperaturmessung (°C) | | | | | | | | |
|---|---|---|---|---|---|---|---|---|---|---|
| | | 23.08. | 24.08. | 25.08. | 26.08. | 06.09. | 07.09. | 08.09. | 0.9.09. | 21.09. |
| | | Datum der Injektionen | | | | | | | | |
| | | | 24.08. | | | | 07.09. | | | |
| 1 | IAD PPV 101 | 38,9 | 39,4 | 39,2 | .39,2 | 38,9 | 39,2 | 38,9 | 39,0 | 39,3 |
| 2 | | 38,9 | 39,2 | 39,3 | 39,1 | 39,1 | 39,3 | 39,0 | 39.0 | 39,0 |
| durchschnittliche Werte | | 38,9 | 39,3 | 39,25 | 39,15 | 39,0 | 39,25 | 38,95 | 39,0 | 39,15 |
| 3 | 010599 | 38,9 | 39,6 | 39,1 | 38,9 | 39,4 | 39,7 | 39,1 | 39,1 | 39,5 |
| 4 | | 39,0 | 38,9 | 39,3 | 39,2 | 39,4 | 39,9 | 39,0 | 39,2 | 39,4 |
| durchschnittliche Werte | | 38,95 | 39,25 | 39,2 | 39,05 | 39,4 | 39,8 | 39,05 | 39,15 | 39,45 |

### 7.3. Beurteilung des Allgemeinzustandes und der Futteraufnahme der Absatzferkel

| Tier-No. | Vakzine | Datum der Injektionen | | | | | | | | | | | | | |
|---|---|---|---|---|---|---|---|---|---|---|---|---|---|---|---|
| | | | 24.0 8 | | | | | | | 07.0 9 | | | | | |
| | | Klinische Visite (Allgemeinznstand / Futteraufnahme) | | | | | | | | | | | | | |
| | | 23.0 8 | 24.0 8 | 25.0 8 | 26.0 8 | 27.0 8 | 28.0 8 | 29.0 8 | 06.0 9 | 07.0 9 | 08.0 9 | 09.0 9 | 10.0 9 | 20.0 9 | 21.0 9 |
| 1 | IAD PPV - 101 | 0/0 | 0/0 | 0/0 | 0/0 | 0/0 | 0/0 | 0/0 | 0/0 | 0/0 | 0/0 | 0/0 | 0/0 | 0/0 | 0/0 |
| 2 | | 0/0 | 0/0 | 0/0 | 0/0 | 0/0 | 0/0 | 0/0 | 0/0 | 0/0 | 0/0 | 0/0 | 0/0 | 0/0 | 0/0 |
| 3 | 01 | 0/0 | 0/0 | 0/0 | 0/0 | 0/0 | 0/0 | 0/0 | 0/0 | 0/0 | 0/0 | 0/0 | 0/0 | 0/0 | 0/0 |
| 4 | 0599 | 0/0 | 0/0 | 0/0 | 0/0 | 0/0 | 0/0 | 0/0 | 0/0 | 0/0 | 0/0 | 0/0 | 0/0 | 0/0 | 0/0 |

0 .... physiologischer Allgemeinzustand und Futteraufnahme
1 .... leichte Beeinträchtigung des Allgemeinzustandes und der Futteraufnahme
2 .... mittlere Beeinträchtigung des Allgemeinzustandes und der Futteraufnahme
3 .... starke Beeinträchtigung des Allgemeinzustandes und der Futteraufnahme

### 9. Diskussion

Absatzferkel, die mit den Vakzinen IAD PPV-101 (diese enthält das neue Adjuvans) und VM 01 0599 (diese enthält das gleiche Antigen, aber als Adjuvans Aluminiumhydroxid) zweimal i.m. immunisiert wurden, zeigten nach den Applikationen keine lokalen und / oder klinischen Symptome, die mit den Impfungen bzw. mit den Impfstoffen in Zusammenhang standen.

An den Injektionsstellen konnten keine lokalen Reaktionen beobachtet werden.

Die Tiere, die mit der Versuchsvakzine IAD PPV-101 immunisiert wurden zeigten ebenfalls wie die Tiere, die mit der Vergleichsvakzine VM 01 0599 immunisiert wurden, nach den Impfungen keinen signifikanten Anstieg der rektalen Körpertemperatur.

Weiterhin zeigten alle Tiere während des Versuchszeitraumes einen physiologischen Allgemeinzustand und Futteraufnahme.

Insgesamt wird festgestellt, daß eine zweimalige i.m. Applikation (ID = 2 ml) des Impfstoffes IAD PPV-101, welcher das neu entwickelte Adjuvans enthält, in Absatzferkeln sicher ist und zu keinen unerwünschten Nebenwirkungen führt.

### 5.1 Wirksamkeitsprüfungen mit Meerschweinchen

### Versuch - Nr. 299/99

### 1. Titel: Versuch-Nr. 299/99 Untersuchung der Verträglichkeit und Wirksamkeit eines neuen Adjuvants : an Meerschweinchen

### 2. Detailierte Beschreibung des Versuches

Methode: - randomisierter Versuch
   - Nachweis des Impferfolges am Labortier mittels serologischer Methode (HAH)
Material : - Impfstoffpräparationen
**Tiere :** - SPF-Meerschweinchen (Charles River)
   Name: Hartley MSW, CRL : (HA)BR, weiblich
   Anzahl : 12
   Alter : 250-300g
Frei von relevanten Keimen : SPF-Zertifikat
Vakzinationsdosis : 0,5 ml / Tier und Applikation
Applikation: s. c.
Immunisierung: 2 x im Abstand von 14 Tagen
Beobachtungszeit : 11 Tage post vacc.
Bewertungskriterium : - Verträglichkeit (systemisch, lokal)
   - PPV-Antikörpertiter (HAH)

### 3. Kontrolltiere

### 2 Meerschweinchen wird jeweils PBS verabreicht.

### 4. Ergebnisse

| **Gruppen-Nr.** | **Impfstoff** | **Antigengehalt (PPV)** | **Adjuvant** | **Anzahl der Meerschweinchen** | **PPV-Antikörpertiter (HAH)** |
|---|---|---|---|---|---|
| 1 | IAD PPV 101 (G98 V04-101/PPV) | 5120 HAE | LAHneu | 3 | 1:512, 1:512, 1:32 |
| 2 | VM 01 0599 | 10240 HAE | AL-hydroxid | 5 | 1:512, 1:1024, 1:1024, 1:256, 1:512 |
| 3 | VM 010697 | 5120 HAE > 50 U.I E.rh. | AL-hydroxide | 2 | 1:256,1:256 |
| 4 | PBS | - | - | 2 | 1:32,1:32 |

| | | | | | |
|---|---|---|---|---|---|
| Durchschnittliche Gruppentiter : Gruppe 1 1 :2^{9*} (*ein Tier war ein serologischer "none responser") Gruppe 2 1:2^{9,2} Gruppe 3 1:2⁸ Gruppe 4 1:2⁵ | | | | | |

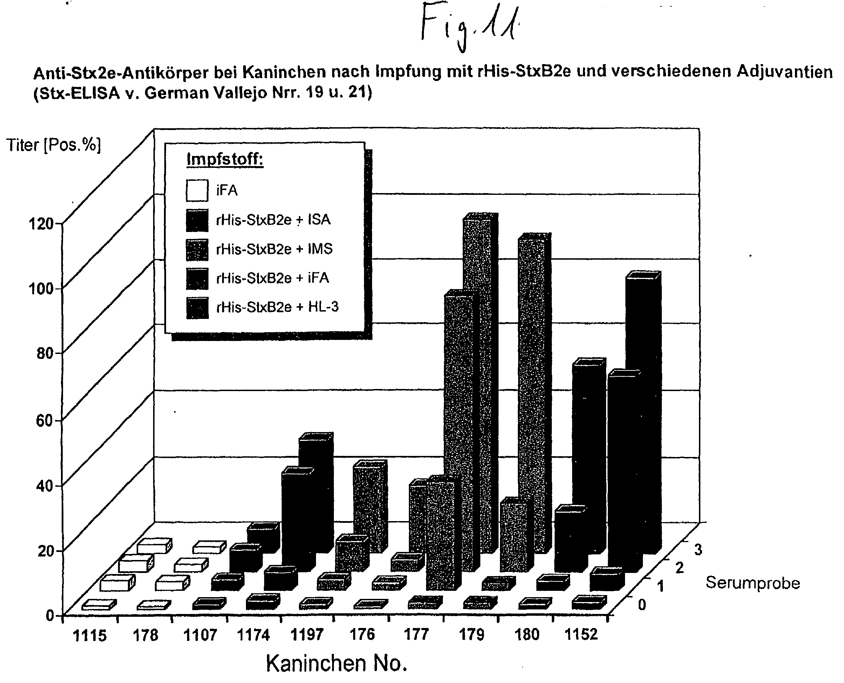

Fig. 10 zeigt die Antikörpertiter der verschiedenen Impfstoffe

### 5. Unerwünschte Nebenwirkungen

- leichte Schwellung an der Injektionstelle bei den Tieren der Gruppe 1, 2 und 3

### 6. Während des Versuches verabreichte Medikamente

- keine

### 7. Diskussion der Ergebnisse

Eine zweimalige s.c. Applikation der verschiedenen Impfstoffmuster an Meerschweinchen führte zu einem deutlichen PPV-Antikörpertiteranstieg im Vergleich zu den Kontrolltieren.
Aus den Ergebnissen der im HAH untersuchten Seren geht hervor, dass sich tendenziell bessere PPV-Antikörpertiter mit der Impfstoffzubereitung - bei gleicher Antigenmenge von 5120 HAE (Versuchsgruppen 1 und 3) - erzielen lassen, die das neu beschriebene Adjuvant enthält. In dieser Gruppe war jedoch ein Tier, welches als serologischer "none responser" gewertet wird, da dieser PPV-Antikörpertiter 14 Tage nach der 2. Impfstoffapplikation identisch mit denen der Kontrolltiere ist.

### 5.2 Wirksamkeitsprüfungen mit Kaninchen

### Versuch - Nr. rStx-Ka 01/2000

### Titel : Untersuchung der immunologischen Potenz des rStx-IIe-B-Proteins in Kombination mit verschiedenen Adjuvantien

### 1. Zusammenfassung

In diesem Versuch wurden 10 Kaninchen dreimal s.c. geimpft. Die Impfdosis betrug jeweils 2 ml pro Tier und Applikation. Es wurde 5 Vakzinepreparationen eingesetzt.

Die Vakzinen enthielten jeweils das rStx-IIe-B-Protein sowie als Adjuvans incomplettes Freud'sche Adjuvans, 2 unterschiedliche kommerzielle Adjuvantien sowie das neu entwickelte LAH Adjuvant

Blutproben wurden unmittelbar vor den Immunisierungen sowie 5 Wochen nach der 3. Immunisierung genommen und im Elisa auf spezifische Antikörper gegen das rSxt-IIe-B-Protein untersucht.

Die Vakzineformulierungen, die das Freud'sche Adjuvans und das neue LAH-Adjuvans enthielten, induzierten in den Tieren die höchsten rStx-IIe-B-Protein-Antikörpertiter.

### 2. Liste der Abkürzungen

- AK: - Antikörper
- rStx-IIe-B-subunit: - rekombinante Shiga-toxin-II_{edema}-B-Subunit
- s.c.: - subcutan

### 3. Materialien und Methoden

### 3.1. Tiere

Spezies : Kaninchen, New Zealand White
Tierkategory : erwachsen
Geschlecht: weiblich
Alter zu Versuchsbeginn : 1600 - 2000 g
Immunstatus der Tiere am Versuchsbeginn : Stx-IIe-AK-negative
Identifikation der Tiere : Ohrmarken

### 3.2. Beschreibung der Impfchargen

| **Chargen-Nr.** | **Gehalt an aktiven Inhaltsstoffens** | **Adjuvans** | **11D** |
|---|---|---|---|
| G97V27-17 | 318,3 µg rStx-IIe-B / ID (1 ml Antigen + 1ml Adjuvans) | LAH (A) * | 1 ID = 2 ml |
| G97V27-16 | | Adjuvans B | |
| G97V27-15 | | Adjuvans C | |
| G97V27-14 | | inkomplette Freund'sche Adjuvans, (D) | |
| G97V27-12 | - | inkomplette Freund'sche Adjuvans (E) | |

| | | | |
|---|---|---|---|
| * Vakzine enthält als Adjuvanz: Isopropylmyristat Emulgator Aerosil R 974 | | | |

### 3.3. Methode

### 3.3.1. Tierhaltung

3.3.1.1. Haltungsbedingungen : Käfighaltung mit Einstreu
3.3.1.2. Futter : Trockenfutter (Altromin)
3.3.1.3. Futterzusätze : keine
3.3.1.4. Wasserversorgung : Trinkwasser (über Nippeltränkflasche)

### 3.3.2. Impfstoffverabreichung

3.3.2.1. Vakzination
3.3.2.1.I . Dosis : 2 ml / Kaninchen und Impfung
3.3.2.1.2. Impfzeitpunkte : 1. Impfung: 10.02.2000
   2. Impfung : 08.03.2000
   3. Impfung : 29.03.2000
3.3.2.1.3. Applikationsweg : parenterally
3.3.2.1.4. Applicationstyp : s.c.
3.3.2.1.5. Vorbehandlung der Vakzine : Schütteln vor Benutzung
3.3.2.1.6. Vorbehandlung der Tiere: keine
3.3.2.1.7. Anzahl der geimpften und der Kontrolltiere : 8 + 2 Kontrolltiere (Placebo)
3.3.2.1.8. Dauer der Beobachtung : 84 Tage

**Tabelle 1 : Überblick über die Tiergruppen**

| **Gruppen-Nr.** | **Nr. der Tiere** | **Vakzination** | | | | | | |
|---|---|---|---|---|---|---|---|---|
| | | **Vakzine** | **1. Vakzination** | | **2. Vakzination** | | **3. Vakzination** | |
| | | | **Dosis/ Tier** | **Applikation** | **Dosis/ Tier** | **Applikation** | **Dosis/ Tier** | **Applikation** |
| 1 | 0180 1152 | G97V27-17 | 2ml | s.c. | 2 ml | s.c. | 2 ml | s.c. |
| 2 | 1107 1174 | G97V27-16 | | | | | | |
| 3 | 1197 0176 | G97V27-15 | | | | | | |
| 4 | 0177 0179 | G97V27-14 | | | | | | |
| 5 (Kontrolltiere) | 1115 0178 | G97V27-12 (Placebo) | | | | | | |

### 3.3.3. Untersuchungsmethoden

### 3.3.3.1. Tägliche Visite

Beurteilung des Allgemeinbefindens und der Futteraufnahme

### 3.3.3.2. Serologische Untersuchungen

Blutentnahmezeiten : 1.0- Probe - vor der 1. Immunisierung
2.1. Probe - vor der 2. Immunisierung
3. 2. Probe - vor der 3. Immunisierung
4. 3. Probe - 5 Wochen post 3.immun.

Die Blutproben wurden mittels eines Elisa auf den in den Tieren induzierten Stx-IIe-B-Antikörpertiter untersucht.

### 4. Versuchsverlauf

### 4.1. Anzahl der Tiere, die vor Versuchsende ausschieden :

- keine

### 4.2. Auftreten von Erkrankungen während des Versuchszeitraumes :

- keine

### 4.3. Behandlung mit anderen Arzneimitteln :

- keine

### 5. Ergebnisse

Die Ergebnisse sind in der Anlage 1 dargestellt sowie in Fig. 11.

### 6. Diskussion

Die Ergebnisse demonstrieren, dass in Abhängigkeit der eingesetzten Adjuvantien bei gleichem Antigengehalt pro Impfdosis unterschiedliche Stx-IIe-AK-Titer in den Kaninchen induziert wurden.

Die höchsten Stx-IIe-B-AK-Titer wurden in den Kannichen gebildet, die mit Vakzinen geimpft wurden, die das inkomplete Freud'sche Adjuvans bzw. das neue LAH-Adjuvans enthielten.

Aber das inkomplete Freud'sche Adjuvans kann aufgrund seiner bekannten Nebenwirkungen nicht als Adjuvans für Vakzine verwendet werden, welche bei den landwirtschaftlichen Nutztiere zur Immunprophylaxe eingesetzt werden.

Das neuentwickelte LAH-Adjuvans kann aufgrund seiner nachgewiesenen Wirksamkeit als potentielles Adjuvans für Impfstoffe für landwirtschaftliche Nutztiere betrachtet werden.

### 7. Anhang

### 7.1. Anhang 1 - Elisa-Untersuchungsergebnisse

### Rekombinante Stx2e-Vakzine: Bestimmung des optimalen Adjuvans

### Experimentelles Design:

1. Impfung von Kaninchen mit rHis-StxB2e und verschiedenen Adjuvantien
2. Blutentnahmen;
3. Sendung von Hm. Lüder vom 11.4.2000 an Dr. Bauerfeind
4. Nachweis von Anti-Stx2e-IgG mittels rHis-StxB2e-ELISA (ELISA No. Stx-19 bis -21, 26)

| *Impfantigen* | *Adjuvans* | *Tier No.* | *Serum- No.* | *Serum- Archiv-No.* | *Titer [Pos.* %*]* | | |
|---|---|---|---|---|---|---|---|
| | | | | | *1:100* | *1:200* | *1:400* |
| [keines] | iFA | 1115 | 0 | R1301/00 | 1,0 | 0,76 | 1,52 |
| | | | 1 | R1302/00 | 3,2 | 2,0 | 1,14 |
| | | | 2 | R1303/00 | 3,3 | 2,0 | 1,91 |
| | | | 3 | R1364/00 | 2,8 | n.t | n.t |
| | | 0178 | 0 | R1304/00 | 0,9 | 0,57 | 0,38 |
| | | | 1 | R1305/00 | 2,7 | 3,34 | 2.48 |
| | | | 2 | R1306/00 | 2,4 | 1.33 | 1,05 |
| | | | 3 | R1360/00 | 1,8 | n.t. | n.t. |
| rHis-StxB2e | G | 1197 | 0 | R1307/00 | 1,5 | 1,43 | 0,38 |
| | | | 1 | R1308/00 | 3,3 | 2,04 | 0,86 |
| | | | 2 | R1309/00 | 9,7 | 6,3 | 4,57 |
| | | | 3 | R1367/00 | 26,5 | n.t. | n.t. |
| | | 0176 | 0 | R1310/00 | 0,8 | 0,55 | 0,68 |
| | | | 1 | R1311/00 | 2,2 | 1,23 | 2,72 |
| | | | 2 | R1312/00 | 3,9 | 2,16 | 1,29 |
| | | | 3 | R1358/00 | 20,8 | n.t. | n.t. |
| rHis-StxB2e | B | 1107 | 0 | R1325/00 | 1,4 | 1,37 | 0,81 |
| | | | 1 | R1326/00 | 3,5 | 2,7 | 2,37 |
| | | | 2 | R1327/00 | 6, 9 | 5.11 | 4,18 |
| | | | 3 | R1363/00 | 7,7 | n.t. | n.t. |
| | | 1174 | 0 | R1316/00 | 2,5 | 2,04 | 1,29 |
| | | | 1 | R1317/00 | 5,8 | 3,15 | 1,48 |
| | | | 2 | R1318/00 | 30,3 | 21,97 | 15,04 |
| | | | 3 | R1366/00 | 34,8 | n.t. | n.t. |
| rHis-StxB2e | iFA | 0177 | 0 | R1313/00 | 1,9 | 0,74 | 0,68 |
| | | | 1 | R1314/00 | 33,5 | 21,41 | 15,71 |
| | | | 2 | R1315/00 | 84.0 | 74.62 | 59.59 |
| | | | 3 | R1359/00 | 102,0 | n.t. | n.t. |
| | | 0179 | 0 | R1298/00 | 2,0 | 2,6 | 2,58 |
| | | | 1 | R1299/00 | 2,1 | 1,24 | 1,33 |
| | | | 2 | R1300/00 | 20, 9 | 13, 38 | 7, 93 |
| | | | 3 | R1361/00' | 95,7 | n.t. | n.t. |
| rHis-StxB2e | (HL-3) L A H (A) | 0180 | 0 | R1319/00 | 1,5 | 1,18 | 1,18 |
| | | | 1 | R1320/00 | 3,1 | 1,81 | 1,81 |
| | | | 2 | R1321/00 | 18,7 | 14,98 | 9.36 |
| | | | 3 | R1362/00 | 57,0 | n.t. | n.t. |
| | | 1152 | 0 | R1322/00 | 2,4 | 1,87 | 1,31 |
| | | | 1 | R1323/00 | 5,5 | 3,68 | 2,68 |
| | | | 2 | R1324/00 | 59,6 | 52,43 | 39,82 |
| | | | 3 | R1365/00 | 83,9 | n.t. | n.t. |

## Patentansprüche

1. Stoffzusammensetzung in Form einer stabilen W/O-Emulsion umfassend
• eine disperse wäßrige Phase,
• eine die disperse wäßrige Phase enthaltende kontinuierliche Ölphase auf der Basis eines flüssigen Fettes und/oder eines flüssigen Öles und/oder eines flüssigen Wachses,
• einen Emulgator und
• hydrophobisiertes, hochdisperses Siliciumdioxid mit einem mittleren Teilchendurchmesser im Nanometerbereich als Stabilisator.

2. Stoffzusammensetzung nach Anspruch 1, bei der die wäßrige Phase auf einer gepufferten Lösung basiert

3. Stoffzusammensetzung nach Anspruch 1 oder 2, die ein Konservierungsmittel umfaßt.

4. Stoffzusammensetzung nach Anspruch 3, bei der das Konservierungsmittel im wesentlichen in der wäßrigen Phase enthalten ist.

5. Stoffzusammensetzung nach einem der Ansprüche 1 bis 4, bei der die Ölphase auf einem Neutralöl und/oder einem Paraffinöl und/oder Isopropylmyristat und/oder Isopropylstearat und/oder Isopropylpalmitat und/oder Ethyloleat und/oder Octylpalmitat und/oder Oleyloleat und/oder Hexyllaurat basiert.

6. Stoffzuramräensetamg nach einem der Ansprüche 1 bis 5, bei der der Emulgator im wesentlichen in der Ölphase enthalten ist

7. Stoffzusammensetzung nach einem der Ansprüche 1 bis 6, bei der der Emulgator ein Polyglycerol und/oder Polyglycerid ist.

8. Stoffzusammensetzung nach einem der Ansprüche 1 bis 7, bei der der Emulgator Polyglyceryl-2Dipolyhydroxistearat ist.

9. Stoffzusannmensetzung nach einem der Ansprüche 1 bis 8, bei der das Siliciumdioxid im wesentlichen in der Ölphase enthalten ist

10. Stoffzusammensetzung nach einem der Ansprüche 1 bis 9, bei der das Siliciumdioxid einen mittleren Teilchendurchmesser von 10 bis 20 Nanometer hat.

11. Stoffzusammensetzung nach einem der Ansprüche 1 bis 10, bei der das Siliciumdioxid an der Kieselsäureoberfläche hydrophobisiert ist.

12. Stoffzuaminensetzung nach einem der Ansprüche 1 bis 11, die bezogen auf 100 ml Emulsion 25 ± 5 ml Isopropylmyristat, 1,5 ± 1,0 ml Polyglyceryl-2Dipolyhydroxistearat, 75 ± 5 ml wäßrige Phase und 1 ± 0,5 g Siliciumdioxid enthält.

13. Stoffzusaminensetzung nach Anspruch 12, bei der der mittlere Tropfendurchmesser der Tropfen der wäßrigen Phase ungefähr 5-30 µm beträgt

14. Stoffzusaminensetzung nach einem der Ansprüche 1 bis 13, bei der die wäßrige Phase mindestens ein Antigen enthält

15. Stoffzusammensetzung nach Anspruch 14, bei der das mindestens eine Antigen einen Volumenanteil der wäßrigen Phase ersetzt.

16. Stoffzusammensetzung nach Anspruch 14 oder 15, bei der das Antigen ein bakterielles, virales oder parasitäres Antigen, ein Pilzantigen, ein Toxoid, ein bakterielles, virales oder parasitäres Spaltantigen, ein DNA-Strang oder -Strangabschnitt oder ein rekombinantes Protein ist.

17. Stoffzusammensetzung nach einem der Ansprüche 14 bis 16, umfassend als Antigen ein Impfantigen für Nutztiere oder Menschen.

18. Stoffzusammensetzung nach einem der Ansprüche 1 bis 17 zur Verwendung als Adjuvans für einen Impfstoff.

19. Stoffzusammensetzung nach Anspruch 18 zur Verwendung als Adjuvans für einen inaktivierten Impfstoff.

20. Stoffzusammensetzung nach Anspruch 18 oder 19, zur Verwendung als Adjuvans für einen Impfstoff für Nutztiere oder Menschen.

21. Stoffzusammensetzung nach einem der Ansprüche 14 bis 17, zur Verwendung als Impfstoff.

22. Stoffzusammensetzung nach Anspruch 21 zur Verwendung als inaktivierter Impfstoff

23. Stoffzusamtnemetzung nach Anspruch 21 oder 22 zur Verwendung als Impfstoff für Nutztiere oder Menschen.

## Claims

1. Material composition in the form of a stable W/O emulsion comprising
• a disperse aqueous phase
• a continuous oil phase containing the disperse aqueous phase based on a liquid fat and/or a liquid oil and/or a liquid wax,
• an emulsifier and
• hydrophobed, highly disperse silicon dioxide as stabiliser with an average particle diameter in the nanometer range.

2. Material composition according to claim 1, in which the aqueous phase is based on a buffered solution.

3. Material composition according to claim 1 or 2, which comprises a preservative.

4. Material composition according to claim 3, in which the preservative is substantially contained in the aqueous phase.

5. Material composition according to any one of claims 1 to 4, in which the oil phase is based on a neutral oil and/or a paraffin oil and/or isopropyl myristate and/or isopropyl stearate and/or isopropyl palmitate and/or ethyl oleate and/or octyl palmitate and/or oleyl oleate and/or hexyl laurate.

6. Material composition according to any one of claims 1 to 5, in which the emulsifier is substantially contained in the oil phase.

7. Material composition according to any one of claims 1 to 6, in which the emulsifier is a polyglycerol and/or polyglyceride.

8. Material composition according to any one of claims 1 to 7, in which the emulsifier is polyglyceryl-2 dipolyhydroxystearate.

9. Material composition according to any one of claims 1 to 8, in which the silicon dioxide is substantially contained in the oil phase.

10. Material composition according to any one of claims 1 to 9, in which the silicon dioxide has a mean particle diameter of 10 to 20 nanometers.

11. Material composition according to any one of claims 1 to 10, in which the silicon dioxide is hydrophobed on the silicic acid surface.

12. Material composition according to any one of claims 1 to 11 which, relative to 100 ml emulsion, contains 25 ± 5 ml isopropyl myristate, 1.5 ± 1.0 ml polyglyceryl-2dipolyhydroxystearate, 75 ± 5ml aqueous phase and 1 ± 0.5 g silicon dioxide.

13. Material composition according to claim 12, in which the mean droplet diameter of the aqueous phase droplets is approximately 5-30 *µ*m.

14. Material composition according to any one of claims 1 to 13, in which the aqueous phase contains at least one antigen.

15. Material composition according to claim 14, in which the at least one antigen replaces a volume fraction of the aqueous phase.

16. Material composition according to claim 14 or 15, in which the antigen is a bacterial, viral or parasitic antigen, a fungal antigen, a toxoid, a bacterial, viral or parasitic split antigen , a DNA strand or strand portion or a recombinant protein.

17. Material composition according to any one of claims 14 to 16, comprising, as an antigen, a vaccination antigen for livestock or humans.

18. Material composition according to any one of claims 1 to 17 for use as an adjuvant for a vaccine.

19. Material composition according to claim 18 for use as an adjuvant for an inactivated vaccine.

20. Material composition according to claim 18 or 19, for use as an adjuvant for a vaccine for livestock or humans.

21. Material composition according to any one of claims 14 to 17 for use as a vaccine.

22. Material composition according to claim 21 for use as an inactivated vaccine.

23. Material composition according to claim 21 or 22 for use as a vaccine for livestock or humans.

## Revendications

1. Composition sous forme d'une émulsion E/H stable comprenant
• une phase aqueuse dispersée
• une phase huileuse continue contenant la phase aqueuse sur la base d'une graisse liquide et/ou d'une huile liquide et/ou d'une cire liquide,
• un émulsifiant et
• du dioxyde de silicium hydrophobisé, très dispersé avec un diamètre moyen de particule à échelle nanométrique comme stabilisant.

2. Composition selon la revendication 1, dans laquelle la phase aqueuse est basée sur une solution tamponnée.

3. Composition selon la revendication 1 ou 2, laquelle comprend un agent de conservation.

4. Composition selon la revendication 3, dans laquelle l'agent de conservation est essentiellement contenu dans la phase aqueuse.

5. Composition selon l'une des revendications 1 à 4, dans laquelle la phase huileuse est basée sur une huile neutre et/ou une huile de paraffine et/ou myristate d'isopropyle et/ou stéarate d'isopropyle et/ou palmitate d'isopropyle et/ou oléate d'éthyle et/ou palmitate d'octyle et/ou oléate d'oléyle et/ou laurate d'hexyle.

6. Composition selon l'une des revendications 1 à 5, dans laquelle l'émulsifiant est essentiellement contenu dans la phase huileuse.

7. Composition selon l'une des revendications 1 à 6, dans laquelle l'émulsifiant est un polyglycérol et/ou polyglycéride.

8. Composition selon l'une des revendications 1 à 7, dans laquelle l'émulsifiant est le polyglyceryl-2 dipolyhydroxystéarate.

9. Composition selon l'une des revendications 1 à 8, dans laquelle le dioxyde de silicium est essentiellement contenu dans la phase huileuse.

10. Composition selon l'une des revendications 1 à 9, dans laquelle le dioxyde de silicium a un diamètre moyen de particule de 10 à 20 nanomètres.

11. Composition selon l'une des revendications 1 à 10, dans laquelle le dioxyde de silicium est hydrophobisé à la surface de l'acide silicique.

12. Composition selon l'une des revendications 1 à 11, laquelle, par rapport à 100 ml d'émulsion, contient 25 ± 5 ml de myristate d'isopropyle, 1,5 ± 1,0 ml de polyglyceryl-2 dipolyhydroxystéarate, 75 ± 5 ml de phase aqueuse et 1 ± 0,5 g de dioxyde de silicium.

13. Composition selon la revendication 12, dans laquelle le diamètre moyen de goutte des gouttes de la phase aqueuse est de 5 à 30 µm environ.

14. Composition selon l'une des revendications 1 à 13, dans laquelle la phase aqueuse contient au moins un antigène.

15. Composition selon la revendication 14, dans laquelle le au moins un antigène remplace une part du volume de la phase aqueuse.

16. Composition selon la revendication 14 ou 15, dans laquelle l'antigène est un antigène bactérien, viral ou parasitaire, un antigène fongique, une toxoïde, un antigène dissocié bactérien, viral ou parasitaire, un brin ou une section de brin d'ADN ou une protéine recombinante.

17. Composition selon l'une des revendications 14 à 16, comprenant comme antigène un antigène vaccinal pour les animaux de rente ou les être humains.

18. Composition selon l'une des revendications 1 à 17 pour l'utilisation comme adjuvant pour un vaccin.

19. Composition selon la revendication 18 pour l'utilisation comme adjuvant pour un vaccin inactivé.

20. Composition selon la revendication 18 ou 19, pour l'utilisation comme adjuvant pour un vaccin destiné aux animaux de rente ou aux être humains.

21. Composition selon l'une des revendications 14 à 17 pour l'utilisation comme vaccin.

22. Composition selon la revendication 21 pour l'utilisation comme vaccin inactivé.

23. Composition selon la revendication 21 ou 22, pour l'utilisation comme vaccin destiné aux animaux de rente ou aux être humains.
